# EUROPEAN PATENT APPLICATION

(11) **EP 4 431 619 A2**
(43) Date of publication of application: **18.09.2024**
(21) Application number: 23213429.6
(22) Date of filing: 30.11.2023
(51) Int. Cl.: C12Q 1/6886

(54) **METHOD OF IDENTIFYING FEATURE OF TEST BODY AND MICRORNA CANCER MARKER**

(30) Priority: 14.03.2023 JP 2023039925
(71) Applicant: Kabushiki Kaisha Toshiba, Tokyo 105-0023 (JP)
(72) Inventor: ANDO, Tomomi, Tokyo (JP); ISHIHARA, Mitsuko, Tokyo (JP); SANO, Yoshitake, Tokyo (JP)
(74) Representative: Marks & Clerk LLP

(57) **Abstract**

According to one arrangement, method of identifying feature of test body is provided. The method includes, measuring a mutation-specific concentration of at least one miRNA contained in the test body, correcting a value of the mutation-specific concentration, and determining whether the test body is a cancer or a non-cancer one using an increase or decrease in the corrected mutation-specific concentration as index.

## Description

### FIELD

The present disclosure relates to a method of identifying the feature of a test body and a microRNA cancer marker.

### BACKGROUND

In general, the association of specific microRNAs (miRNAs) with specific cancers has been studied. There is a possibility that the miRNA considered to be particularly highly relevant can be used as a marker for identifying the feature of a test body. For example, it has been proposed to predict whether or not a target is cancer by specifying the concentration, presence or absence, or the like of such a marker in a test body.

### BRIEF DESCRIPTION OF THE DRAWINGS

FIG. 1 is a flowchart showing an example of an identification method of the first arrangement.
FIG. 2 is a diagram showing an example of mutation according to the first arrangement.
FIG. 3 is a schematic diagram showing a concept of the first arrangement.
FIG. 4 is a flowchart showing a further example of the identification method according to the first arrangement.
FIG. 5 is a flowchart showing another example of the identification method according to the first arrangement.
FIG. 6A is a flowchart showing an example of a process according to the first arrangement.
FIG. 6B is a flowchart showing an example of a process according to the first arrangement.
FIG. 7 is a diagram showing experimental results of Example 2.
FIG. 8 is a diagram showing experimental results of Example 3.
FIG. 9 is a diagram showing experimental results of Example 4.
FIG. 10 is a diagram showing experimental results of Example 5.
FIG. 11 is a diagram showing experimental results of Example 6.
FIG. 12 is a diagram showing experimental results of Example 7.
FIG. 13 is a diagram showing experimental results of Example 8.
FIG. 14 is a diagram showing experimental results of Example 9.
FIG. 15A is a diagram showing experimental results of Example 10.
FIG. 15B is a diagram showing experimental results of Example 11.
FIG. 16A is a diagram showing experimental results of Example 12.
FIG. 16B is a diagram showing experimental results of Example 13.
FIG. 17A is a diagram showing experimental results of Example 14.
FIG. 17B is a diagram showing experimental results of Example 15.
FIG. 18A is a diagram showing experimental results of Example 16.
FIG. 18B is a diagram showing experimental results of Example 17.

### DETAILED DESCRIPTION

According to one arrangement, a method for identifying the feature of a test body using a miRNA is provided. The method includes measuring a mutation-specific concentration of at least one miRNA contained in the test body, correcting a numerical value of the mutation-specific concentration in order to standardize data and acquiring a corrected mutation-specific concentration, and determining whether the test body is a cancer test body or a non-cancer test body using an increase or decrease in the obtained corrected mutation-specific concentration as an index.

According to an aspect of the present invention, it may be an object to provide a method for identifying the feature of a test body and a miRNA cancer marker having stable detection performance.

The present inventors have focused on miRNA mutations in their own research so far, and have studied a method for identifying between cancer subjects and healthy subjects by using them as cancer markers. In the course of the study, it has been found that the tendency of increase/decrease in miRNA concentration between test bodies and/or data sets has a feature that a difference easily occurs and variation is large. It has been found that the performance is not stable due to such a feature. The present inventors have made such a situation a unique problem, and have intensively studied for the purpose of solving the problem, thereby reaching an arrangement proposed below. According to these arrangements, for example, a difference is less likely to occur in the tendency of increase or decrease of the miRNA concentration between test bodies and data sets, the variation is smaller, and the performance is stabilized, so that it is possible to provide a technology that can be used widely.

### (First Arrangement)

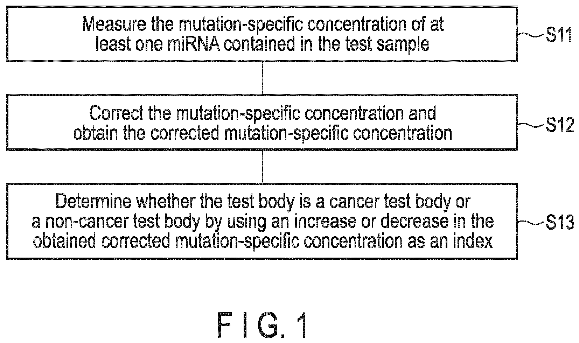

In an example of a method for identifying the feature of a test body according to the first arrangement, as illustrated in FIG. 1, the method for identifying feature of a test body using a miRNA according to the arrangement includes measuring a mutation-specific concentration of at least one miRNA contained in the test body, correcting a numerical value of the mutation-specific concentration in order to standardize data and acquiring a corrected mutation-specific concentration, and determining whether the test body is a cancer test body or a non-cancer test body using an increase or decrease in the obtained corrected mutation-specific concentration as an index. By identifying the feature of a test body, for example, it is possible to identify whether the test body is derived from a cancer subject or a healthy subject or a subject not suffering from the cancer in question, that is, a non-cancer subject.

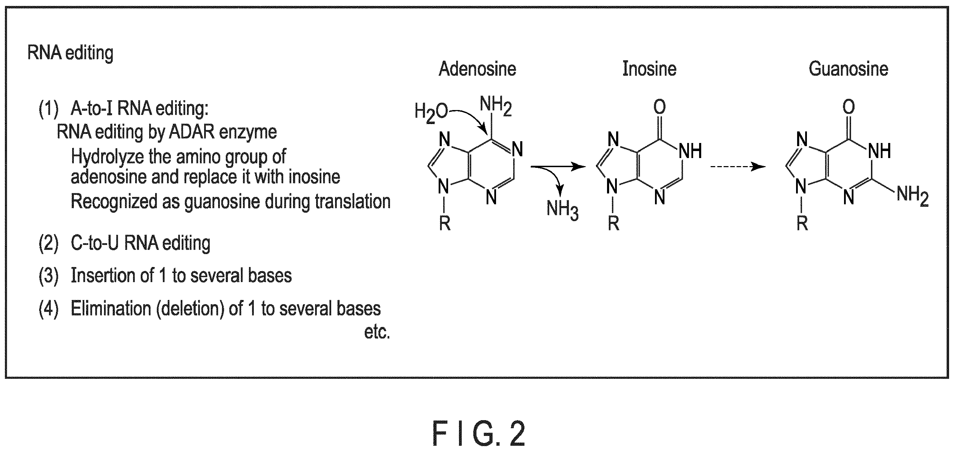

The mutation referred to herein may be, for example, a single nucleotide polymorphism and RNA editing. RNA editing is a mechanism in animals and plants in which a base sequence of RNA transcribed from DNA or RNA being transcribed is replaced, one to several bases are inserted, or eliminated. It is also considered as one of post-RNA transcription modifications and has been reported to be involved in the control of various biological processes. A typical RNA editing example will be described with reference to FIG. 2. That is, typical examples of RNA editing include (1) A-to-I RNA editing, (2) C-to-U RNA editing, (3) insertion of 1 to several bases, (4) elimination (deletion) of 1 to several bases, and the like. A-to-I RNA editing (adenosine to inosine editing) is RNA editing with an ADAR enzyme. The amino group of adenosine (A) is hydrolyzed to be substituted with inosine (I), and is recognized as guanosine (G) having a similar chemical structure at the time of translation. C-to-U RNA editing (cytidine to uridine editing) is one in which cytidine (C) is replaced with uridine (U). In the analysis method, regardless of the mechanism of RNA editing, RNA having a sequence variation may be regarded as a mutation in light of the reference sequence. A preferred mutation is not a sequence variation due to diversity (SNP) on DNA, but is a mutation that occurred after RNA transcription, but it is not necessary to prove that the mutation occurred after RNA transcription.

Here, "correcting the numerical value of the mutation-specific concentration" means correcting the numerical value of the mutation-specific concentration in order to standardize the data, and is correction performed in order to enable general-purpose comparison for mutually different test bodies. Thereby, individual differences of unintended data other than the intended mutation included in the individual data are excluded for standardization. Although specifically described later, such standardization may be arbitrarily selected according to the analysis method used. For example, in a case where the miRNA is comprehensively analyzed, correction may be performed so as to exclude the influence of the size of the data amount.

Here, the "test body" is used interchangeably with "sample" and "test sample", and is a target substance collected from a subject or a test animal and to be analyzed.

According to the first arrangement, it is possible to improve versatility by comparing mutation-specific concentrations. Such a method is less likely to be affected by a difference in miRNA extraction efficiency, a difference in tendency of increase/decrease in concentration, and variations among test bodies, which can occur among test bodies and data sets, and thus stable performance can be obtained.

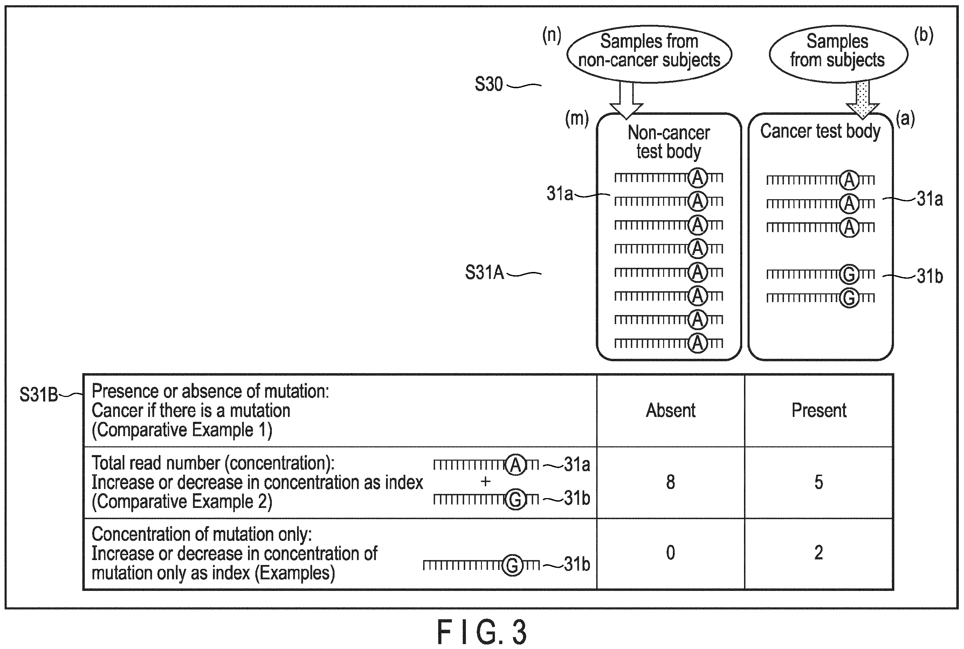

Although details will be described later, an outline of the first arrangement will be described using an exemplary model with reference to FIG. 3. In S31A, the cancer test body (a) and the non-cancer test body (m) are described together. The cancer test body (a) relates to a specific miRNA, and includes a wild type 31a and a mutant type 31b. On the other hand, the non-cancer test body (m) relates to a specific miRNA and includes wild type 31a. In this case, for convenience, there are mainly three ways of determining the feature of a test body, that is, whether the test body is a cancer test body or a non-cancer test body, using the mutation as an index.

The first method is a method using the presence or absence of mutation as an index as shown in S31B. That is, when a mutation is detected, it is determined to be a cancer test body, and when no mutation is detected, it is determined to be a non-cancer test body. The second method is a method in which the total number of reads in a test body, that is, the increase or decrease in the concentration of the miRNA in question is used as an index. The third (first arrangement) is a method for determining by increasing or decreasing the concentration of only mutation. In the case of the first method and the second method, there is a possibility of being affected by the presence of the wild type contained in the test body or the amount of miRNA in question, that is, the number of reads. Therefore, the obtained results are likely to vary depending on each test body. On the other hand, by focusing only on mutation, measuring the number of reads of mutated one of at least one type of miRNA, that is, the concentration (mutation-specific concentration), and correcting the number of reads by the number of reads of the entire test body, and using thus normalized number of reads as an index, it is possible to suppress variation between samples and obtain a stable result.

Examples of the miRNA having a mutation used in the first arrangement include, but not limited to, SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, and SEQ ID NO: 13. For example, preferred miRNAs having mutations are SEQ IN NO: 1, SEQ ID NO: 2, SEQ ID NO: 3, SEQ ID NO: 4, SEQ ID NO: 5, SEQ ID NO: 6, SEQ ID NO: 7 and SEQ ID NO: 8 (Table 1).

**Table 1: Cancer markers**

| No. | miRNA_ID_mu | Sequence_mu | Length | AUC values for single markers |
|---|---|---|---|---|
| 1 | 17_hsa-miR-199a-5p_C-T | CCCAGTGTTCAGACTATCTGTTC | 23 | 0.777 |
| 2 | 9_hsa-miR-1260b_A-G | ATCCCACCGCTGCCACCAT | 19 | 0.773 |
| 3 | 18_hsa-miR-146b-5p_A-G | TGAGAACTGAATTCCATGGGCTG | 23 | 0.766 |
| 4 | 9_hsa-miR-33b-5p_C-T | GTGCATTGTTGTTGCATTGC | 20 | 0.764 |
| 5 | 20_hsa-miR-15b-5p_A-G | TAGCAGCACATCATGGTTTGCA | 22 | 0.760 |
| 6 | 10_hsa-miR-92b-3p_C-T | TATTGCACTTGTCCCGGCCTCC | 22 | 0.746 |
| 7 | 12_hsa-miR-106a-5p_C-T | AAAAGTGCTTATAGTGCAGGTAG | 23 | 0.712 |
| 8 | 11_hsa-miR-98-5p_A-G | TGAGGTAGTAGGTTGTATTGTT | 22 | 0.705 |
| 9 | 13_hsa-miR-130a-3p_A-G | CAGTGCAATGTTGAAAGGGCAT | 22 | 0.689 |
| 10 | 11_hsa-miR-26a-5p_C-T | TTCAAGTAATTCAGGATAGGCT | 22 | 0.687 |
| 11 | 16_hsa-let-7d-5p_C-T | AGAGGTAGTAGGTTGTATAGTT | 22 | 0.644 |
| 12 | 17_hsa-let-7g-5p_A-G | TGAGGTAGTAGTTTGTGCAGTT | 22 | 0.614 |
| 13 | 9_hsa-miR-501-3p_C-T | AATGCACCTGGGCAAGGATTCT | 22 | 0.601 |

In the present specification, "T (thymine)" written for convenience for all sequences may be "U". In practice, the expression "T" is intended to include both cases where the site in question is "U (uracil)" and where it is "T (thymine)". Both the case where the nucleobase at the site in question is "U (uracil)" and the case where the nucleobase at the site in question is "T (thymine)" are equally within the scope of rights. For example, when RNA is analyzed for a subject, RNA is generally converted into DNA by cDNA synthesis and analyzed. For example, when each sequence is described as an RNA sequence, the nucleobase at the site in question is expressed as "U", and when described as a DNA sequence, the nucleobase at the site in question is expressed as "T". Therefore, here: when expressed as miRNA present in serum, the point in question is "U"; one extracted from the test body is "U" because it is RNA; and since the sequence amplified based on the cDNA obtained by reverse transcription is a DNA sequence, the point in question is "T".

Hereinafter, in order to avoid confusion, all base sequences are denoted as DNA for convenience. Therefore, in terms of notation, the point in question is described as "T". From such circumstances, as described above, what is included in the scope of right as an arrangement is equally included for a sequence in which the nucleobase at the site in question is "U (uracil)" and a sequence in which the nucleobase is "T (thymine)".

### (Second Arrangement)

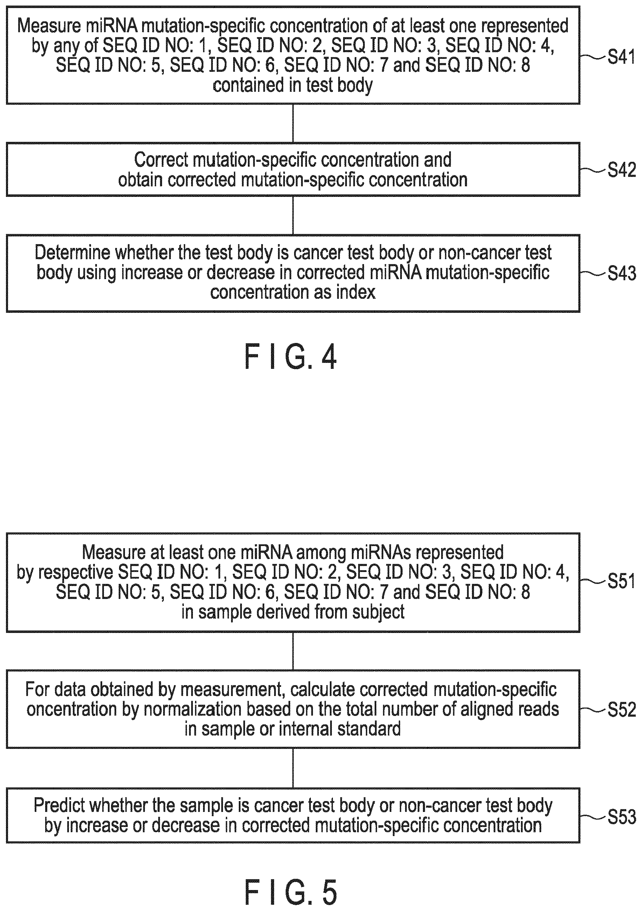

A second arrangement is the method according to the first arrangement, in which a miRNA group having a mutation as described above is used as a miRNA cancer marker set. For example, the example of the first arrangement using such a marker set includes, as shown in FIG. 4, measuring the miRNA mutation-specific concentration of at least one represented by any one of SEQ ID NO: 1, SEQ ID NO: 2, SEQ ID NO: 3, SEQ ID NO: 4, SEQ ID NO: 5, SEQ ID NO: 6, SEQ ID NO: 7, and SEQ ID NO: 8 contained in the test body (S41), correcting the mutation-specific concentration and obtaining the corrected mutation-specific concentration (S42), and determining whether the test body is a cancer test body or a non-cancer test body using an increase or decrease in the obtained miRNA mutation-specific concentration as an index (S43).

**Table 2: Cancer markers**

| No. | miRNA_ID_mu | Sequence_mu | Length | AUC values for single markers |
|---|---|---|---|---|
| 1 | 17_hsa-miR-199a-5p_C-T | CCCAGTGTTCAGACTATCTGTTC | 23 | 0.777 |
| 2 | 9_hsa-miR-1260b_A-G | ATCCCACCGCTGCCACCAT | 19 | 0.773 |
| 3 | 18_hsa-miR-146b-5p_A-G | TGAGAACTGAATTCCATGGGCTG | 23 | 0.766 |
| 4 | 9_hsa-miR-33b-5p_C-T | GTGCATTGTTGTTGCATTGC | 20 | 0.764 |
| 5 | 20_hsa-miR-15b-5p_A-G | TAGCAGCACATCATGGTTTGCA | 22 | 0.760 |
| 6 | 10_hsa-miR-92b-3p_C-T | TATTGCACTTGTCCCGGCCTCC | 22 | 0.746 |
| 7 | 12_hsa-miR-106a-5p_C-T | AAAAGTGCTTATAGTGCAGGTAG | 23 | 0.712 |
| 8 | 11_hsa-miR-98-5p_A-G | TGAGGTAGTAGGTTGTATTGTT | 22 | 0.705 |

SEQ ID NO: 1 has a miRNA ID of "17_hsa-miR-199a-5p_C-T" and a structure in which the 17th C (cytosine) is mutated to T (thymine) from the 5' side of the wild type "hsa-miR-199a-5p". The sequence is "5' CCCAGTGTTCAGACTATCTGTTC 3'" (23 bases long). SEQ ID NO: 2 has a miRNA ID of "9_hsa-miR-1260b_A-G", and a structure in which the ninth A (adenine) is mutated to G (guanine) from the 5' side of the wild type "hsa-miR-1260b". The sequence is "5' ATCCCACCGCTGCCACCAT 3'" (19 bases long). SEQ ID NO: 3 has a miRNA ID of "18_hsa-miR-146b-5p_A-G", and a structure in which the 18th A (adenine) is mutated to G (guanine) from the 5' side of the wild type "hsa-miR-146b-5p". The sequence is "5' TGAGAACTGAATTCCATGGGCTG 3'" (23 bases long). SEQ ID NO: 4 has a miRNA ID of "9_hsa-miR-33b-5p_C-T" and a structure in which the ninth C (cytosine) is mutated to T (thymine) from the 5' side of the wild type "hsa-miR-33b-5p". The sequence is "5' GTGCATTGTTGTTGCATTGC 3'" (20 bases long). SEQ ID NO: 5 has a miRNA ID of "20_hsa-miR-15b-5p_A-G", and a structure in which the 20th A (adenine) is mutated to G (guanine) from the 5' side of the wild type "hsa-miR-15b-5p". The sequence is "5' TAGCAGCACATCATGGTTTGCA 3'" (22 bases long). SEQ ID NO: 6 has a miRNA ID of "10_hsa-miR-92b-3p_C-T" and a structure in which the 10th C (cytosine) is mutated to T (thymine) from the 5' side of the wild type "hsa-miR-92b-3p". The sequence is "5' TATTGCACTTGTCCCGGCCTCC 3'" (22 bases long). SEQ ID NO: 7 has a miRNA ID of is "12_hsa-miR-106a-5p_C-T" and a structure in which the 12th C (cytosine) is mutated to T (thymine) from the 5' side of the wild type "hsa-miR-106a-5p". The sequence is "5' AAAAGTGCTTATAGTGCAGGTAG 3'" (23 bases long). SEQ ID NO: 8 has a miRNA ID of "11_hsa-miR-98-5p_A-G", and a structure in which the 11th A (adenine) is mutated to G (guanine) from the 5' side of the wild type "hsa-miR-98-5p". The sequence is "5' TGAGGTAGTAGGTTGTATTGTT 3'" (22 bases long).

According to the second arrangement, it is possible to improve versatility by comparing mutation-specific concentrations. By such a method, it is less likely to be affected by a difference in miRNA extraction efficiency, a difference in tendency of increase/decrease in concentration, and variations among test bodies, which can occur among test bodies and data sets, and thus stable performance can be obtained.

### (Third Arrangement)

A third arrangement is a miRNA cancer marker set in which miRNAs having mutations used in the first and second arrangements are used in combination. Examples of the miRNA cancer marker set include, but not limited to, miRNAs each having SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 and SEQ ID NO: 13, or complementary sequences thereof. Alternatively, it consists of miRNAs each having SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 and SEQ ID NO: 13, or complementary sequences thereof. For example, further examples of miRNA cancer marker sets include miRNAs each having SEQ ID NO: 1, SEQ ID NO: 2, SEQ ID NO: 3, SEQ ID NO: 4, SEQ ID NO: 5, SEQ ID NO: 6, SEQ ID NO: 7, and SEQ ID NO: 8, or complementary sequences thereof. Alternatively, it consists of miRNAs each having SEQ ID NO: 1, SEQ ID NO: 2, SEQ ID NO: 3, SEQ ID NO: 4, SEQ ID NO: 5, SEQ ID NO: 6, SEQ ID NO: 7, and SEQ ID NO: 8, or complementary sequences thereof. Alternatively, a miRNA cancer marker may comprise a sequence selected from a group consisting of SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8, and a complementary sequence thereof. In the method for identifying the feature of a test body described above, a mutation-specific concentration can be measured for at least one miRNA in these miRNA cancer marker sets. In addition, each miRNA may include or consist of a sequence represented by any of the above-described SEQ ID NOs. In addition, such a marker may be used as, for example, a cancer marker which is a miRNA including a sequence of SEQ ID NO: 1, SEQ ID NO: 2, SEQ ID NO: 3, SEQ ID NO: 4, SEQ ID NO: 5, SEQ ID NO: 6, SEQ ID NO: 7, or SEQ ID NO: 8, or a complementary sequence thereof, for identifying between cancer subjects and healthy subjects (non-cancer subjects). Furthermore, the sequence may be a sequence in which one or several of the above-described sequences are substituted, deleted, or added. However, the wild type sequence distinguished by the above-described single base substitution is not included. For example, in the case of the miRNA ID "17_hsa-miR-199a-5p_C-T" (SEQ ID NO: 15' CCCAGTGTTCAGACTATCTGTTC 3'), the 17th from the 5' side is "C (cytosine)" in the case of the wild type and "T (thymine)" in the case of the mutant type. That is, one or several substitutions or deletions, or one or several additions may be included in the site excluding the 17th site from the 5' side or the corresponding site. Such features about additional mutations are similar for each of SEQ ID NO: 1 to NO: 20.

Each of the sequences of SEQ ID NO: 1 to NO: 20 described above may be expressed as a miRNA ID, and it is also possible to use such a miRNA indicated as an ID as a marker for cancer detection. Such a marker for cancer detection is 17_hsa-miR-199a-5p_C-T (corresponding to SEQ ID NO: 1), 9_hsa-miR-1260b_A-G (corresponding to SEQ ID NO: 2), 18_hsa-miR-146b-5p_A-G (corresponding to SEQ ID NO: 3), 9_hsa-miR-33b-5p_C-T (corresponding to SEQ ID NO: 4), 20_hsa-miR-15b-5p_A-G (corresponding to SEQ ID O: 5), 10_hsa-miR-92b-3 p_C-T (corresponding to SEQ ID NO: 6), 12_hsa-miR-106a-5p_C-T (corresponding to SEQ ID NO: 7), 11_hsa-miR-98-5p_A-G (corresponding to SEQ ID NO: 8), 13_hsa-miR-130a-3p_A-G (corresponding to SEQ ID NO: 9), 11_hsa-miR-26a-5p_C-T (corresponding to SEQ ID NO: 10), 16_hsa-let-7d-5p_C-T (corresponding to SEQ ID NO: 11), 17_hsa-let-7g-5p_A-G (corresponding to SEQ ID NO: 12), or 9_hsa-miR-501-3p_C-T (corresponding to SEQ ID NO: 13). These miRNAs may be used at least singly, or in combination of two or more types, or as a set of SEQ ID NO: 1 to NO: 8, or as a set of SEQ ID NO: 1 to NO: 13. The miRNA represented by these IDs may include one or several substitutions or deletions, or one or several additions at a site, or a corresponding site to the site, other than a site that characterizes the wild type and mutant type of each sequence as with each sequence. For example, in the case of 17_hsa-miR-199a-5p_C-T, a site excluding the 17th site from the 5' side or a site corresponding to a site other than the 17th site from the 5' side may contain such a further mutation, one or several substitutions or deletions, or one or several additions.

For instance, the miRNA cancer marker may include a miRNA selected from a group consisting of 17_hsa-miR-199a-5p_C-T, 9_hsa-miR-1260b_A-G, 18_hsa-miR-146b-5p_A-G, 9_hsa-miR-33b-5p_C-T, 20_hsa-miR-15b-5p_A-G, 10_hsa-miR-92b-3p_C-T, 12_hsa-miR-106a-5p_C-T, 11_hsa-miR-98-5p_A-G, 13_hsa-miR-130 a-3p_A-G, 11_hsa-miR-26a-5p_C-T, 16_hsa-let-7d-5p_C-T, 17_hsa-let-7g-5p_A-G and 9_hsa-miR-501-3p_C-T. The miRNA cancer marker may be a miRNA selected from a group consisting of 17_hsa-miR-199a-5p_C-T, 9_hsa-miR-1260b_A-G, 18_hsa-miR-146b-5p_A-G, 9_hsa-miR-33b-5p_C-T, 20_hsa-miR-15b-5p_A-G, 10_hsa-miR-92b-3p_C-T, 12_hsa-miR-106a-5p_C-T, and 11_hsa-miR-98-5p_A-G.

Alternatively, for example, the miRNA cancer marker may be used as a set. The set may be a group of miRNAs each being 17_hsa-miR-199a-5p_C-T, 9_hsa-miR-1260b_A-G, 18_hsa-miR-146b-5p_A-G, 9_hsa-miR-33b-5p_C-T, 20_hsa-miR-15b-5p_A-G, 10_hsa-miR-92b-3p_C-T, 12_hsa-miR-106a-5p_C-T, 11_hsa-miR-98-5p_A-G, 13_hsa-miR-130 a-3p_A-G, 11_hsa-miR-26a-5p_C-T, 16_hsa-let-7 d-5p_C-T, 17_hsa-let-7 g-5p_A-G and 9_hsa-miR-501-3p_C-T. The miRNA cancer marker set may be a group of miRNAs each being 17_hsa-miR-199a-5p_C-T, 9_hsa-miR-1260b_A-G, 18_hsa-miR-146b-5p_A-G, 9_hsa-miR-33b-5p_C-T, 20_hsa-miR-15b-5p_A-G, 10_hsa-miR-92b-3p_C-T, 12_hsa-miR-106a-5p_C-T, and 11_hsa-miR-98-5p_A-G.

According to the third arrangement, it is possible to improve versatility by comparing mutation-specific concentrations. By using such a marker, it is less likely to be affected by a difference in miRNA extraction efficiency, a difference in tendency of increase/decrease in concentration, and variations among test bodies, which can occur among test bodies and data sets, and thus stable performance can be obtained.

### (Fourth Arrangement)

As shown in FIG. 5, the method for identifying the feature of a test body according to the fourth arrangement includes: measuring at least one miRNA of miRNAs respectively having SEQ ID NO: 1, SEQ ID NO: 2, SEQ ID NO: 3, SEQ ID NO: 4, SEQ ID NO: 5, SEQ ID NO: 6, SEQ ID NO: 7, or SEQ ID NO: 8 or complementary sequences thereof in a test body derived from a subject (S51); calculating a corrected mutation-specific concentration of data obtained by the measurement by normalization based on the total number of aligned reads in the test body or an internal standard (S52); and predicting whether the test body is a cancer test body or a non-cancer test body by increase or decrease in the corrected mutation-specific concentration (S53). Here, the "total number of aligned reads" refers to the amount of all miRNAs contained in a sample containing both variants and wild type miRNAs, that is, the total number of reads aligned to the sequence of the miRNAs, with respect to the miRNA. In addition, the miRNA(s) of interest may be a miRNA represented by SEQ ID NO: 1 to 13, that is, miRNAs respectively having a sequence consisting of or including each of SEQ ID NO: 1 to NO: 13, at least one type of miRNA respectively including one of them, a combination of two or more types of miRNAs respectively including one of them, or all types of miRNAs respectively including one of SEQ ID NO: 1 to 13. The calculation of the mutation-specific concentration by normalization based on the total number of aligned reads in the test body can be calculated by multiplying the TMM value of each miRNA of interest including both the mutant and the wild type by the obtained mutation appearance frequency information, as described later. The internal standard may be any miRNA selected according to a predetermined standard, and for example, a miRNA that is stably expressed without being affected by the feature of a test body and is suitable for quantitatively correcting the target of interest, such as a miRNA having a high abundance such as hsa-miR-486-5p, can be selected, but the example is not limited thereto. It can be normalized by being divided by the internal standard.

By determining whether the test body is a cancer test body or a non-cancer test body, the information obtained can be used as a material by which a doctor determines whether a subject that is an origin of the test body has a possibility of suffering from cancer, a possibility of suffering from cancer from now on, whether the subject is at risk of developing cancer, or whether the subject does not have cancer. For example, when a doctor performs diagnosis using the method of the arrangement, a method of identifying the feature of a test body using miRNA can also be used as a diagnosis method. In this case, for example, the present invention can be provided as a method for identifying between a cancer subject and a healthy subject, including measuring a mutation-specific concentration of at least one miRNA contained in a test body, correcting the mutation-specific concentration to obtain a corrected mutation-specific concentration, and determining whether the test body is a cancer test body or a non-cancer test body using an increase or decrease in the obtained corrected mutation-specific concentration as an index. In that case, the method can include measuring a mutation-specific concentration of at least one miRNA contained in the test body, correcting the mutation-specific concentration to obtain a corrected mutation-specific concentration, and determining the possibility that the subject has cancer or the risk of developing cancer using an increase or decrease in the obtained corrected mutation-specific concentration as an index.

In the present specification, cancer includes cancer of any stage, and includes, for example, a state in which cancer remains in an organ in an origin, a state in which cancer has further spread to a surrounding tissue, a state in which cancer has further metastasized to a lymph node, a state in which cancer has metastasized to a further distant organ, and the like. In addition, in the present specification, breast cancer refers to a malignant tumor (neoplasm) formed in mammary gland tissue. For example, breast cancer also includes what is commonly referred to as "cancer of breast" or "mammary cancer". In addition, the breast cancer according to the arrangement includes any type of breast cancer, for example, lobular breast cancer or ductal breast cancer. In addition, the breast cancer according to the arrangement includes, for example, an epithelial tumor, a non-epithelial tumor, and a malignant lobular tumor including both epithelial and non-epithelial.

For example, the cancer may be at least one cancer selected from the group consisting of breast cancer, colorectal cancer, lung cancer, stomach cancer, pancreatic cancer, cervical cancer, uterine cancer, ovarian cancer, sarcoma, prostate cancer, bile duct cancer, bladder cancer, esophageal cancer, liver cancer, brain tumor, and kidney cancer. In addition, for example, the cancer may be breast cancer and pancreatic cancer.

For correction of the mutation-specific concentration, for example, the obtained frequency information may be multiplied by the TMM value (that is, the normalized value based on the total number of aligned reads) of each miRNA of interest including both the mutant and the wild type. In addition, for example, by normalizing the total number of aligned reads by a TMM method, an RPKM normalization method, or the like, and comparing them each other, it is also possible to reflect the measured value, that is, the measured mutation-specific concentration of at least one miRNA contained in the test body, and correct the measured concentration. This makes it possible to universally compare a plurality of miRNAs. For the analysis, a method of simply comparing the number of reads or the normalized value and performing a significant difference test, and a platform for transcriptome analysis such as DESeq, EdgeR and the like, medical statistical software such as EZR (Easy R), JMP (registered trademark) and the like, or methods of creating a discriminant using Fisher's discriminant analysis, nonlinear discriminant analysis based on a Mahalanobis distance, logistic regression analysis, machine learning such as neural network, random forest and the like, can be used, but the method is not limited to these methods. For example, when the NGS method is used, the mutation output software may be used to output the existence ratio of variants (That is, the frequency information), and the TMM value may be multiplied by the frequency information to calculate the corrected mutation-specific concentration. In addition, for example, in the case of using the PCR method, the mutant-specific concentration is measured by a mutation-specific detection (that is, amplification) method, and the corrected mutation-specific concentration can be acquired by a method such as dividing by the miRNA concentration serving as an internal standard, or the like. In other words, for example, the corrected mutation-specific concentration can be obtained by multiplying the normalized value of the total number of aligned reads by the frequency information in the case of NGS, or dividing by the internal standard in the case of PCR.

The test body may be, for example, a body fluid or the like obtained from a subject. For example, the step of measuring the miRNA mutation-specific concentration in the body fluid preferably mainly includes (i) collection of a test body from a subject, (ii) extraction of the miRNA from the test body, and (iii) calculation of a concentration specific to the miRNA in which sequence variation has occurred, and typical methods thereof are described below, but are not limited thereto.

### (i) Test body collection from subject

The test body used for the measurement is collected from a subject, and is not particularly limited, and for example, blood, serum, plasma, white blood cells, urine, digestive fluid, saliva, gastric fluid, sweat, tears, nasal mucus, semen, vaginal fluid, amniotic fluid, milk, lymph fluid, tissue, intraoral mucous membrane, sputum, and the like can be used. The test body is subjected to processing such as centrifugation, precipitation, extraction and/or separation and the like, and brought into a state suitable for amplification of nucleic acid. In addition, when the collected test body is suitable for amplification of nucleic acid as it is, the collected sample may be used as it is as a test body.

### (ii) Extraction of miRNA from test body

The extraction of the nucleic acid can be performed using, but not limited to, a commercially available nucleic acid extraction kit such as NucleoSpin (registered trademark) miRNA Plasma (manufactured by Takara Bio Inc.), Quick-cfRNA Serum & Plasma Kit (manufactured by Zymo Research Inc.), miRNeasy Serum/Plasma kit (manufactured by Qiagen), miRVana PARIS isolation kit (manufactured by Thermo Fisher Scientific Inc.), PureLinkTM Total RNA Blood Kit (manufactured by Thermo Fisher Scientific Inc.), Plasma/Serum RNA Purification Kit (manufactured by Norgen Biotech Inc.), microRNA Extractor (registered trademark) SP Kit (manufactured by Wako Pure Chemical Industries, Ltd.), High Pure miRNA Isolation Kit (manufactured by Sigma-Aldrich Co.), or the like. In addition, regardless of the kit, it is also possible to use a simple method in which a test body is diluted with a buffer and then centrifuged after heat treatment at 80 to 100°C to obtain a supernatant.

### (iii) Calculation of miRNA-specific concentration at which sequence variation has occurred

The step of quantifying the miRNA mutation-specific concentration can be performed using a general method for quantifying RNA, particularly short-chain RNA such as miRNA and the like, by designing primers, probes, and the like used for detection with a sequence specific to mutation. Although the method is not limited, for example, the miRNA is reverse-transcribed to generate cDNA, the obtained cDNA is amplified, and the amplification product can be detected and quantified. In a case where the RNA is short, in order to facilitate amplification, it is also generally performed to extend the cDNA obtained by reverse transcription so as to add an artificial sequence to the end of the cDNA. In addition, a rolling circle amplification method is known as a technique for directly amplifying RNA in a test body without performing reverse transcription, and detecting and quantifying an amplification product. For the amplification, for example, a PCR method, a qPCR method, or a LAMP method can be used. Detection and quantification may occur after amplification or over time during amplification. In addition, it may be performed in combination with a microarray method.

For the detection and quantification, for example, a measurement method using a signal based on turbidity or absorbance, a measurement method using an optical signal, a measurement method using an electrochemical signal, or a combination thereof and the like can be used. For example, the miRNA can be quantified from the intensity or the amount of change of the signal obtained according to the amount of the amplification product, the time until the signal reaches the threshold (rise time), or the number of cycles (rise cycle number) when the PCR method is used. The quantitative value of the miRNA may be determined using a calibration curve. The abundance of the miRNA may be calculated, for example, as the number of copies of the target miRNA per unit amount of the test body. Such a quantitative method may be performed using a commercially available kit. Examples of the commercially available kit include TaqMan (registered trademark) Advanced miRNA Assays (manufactured by Thermo Fisher Scientific Inc., Catalog No. A25576), miRCURY LNA (registered trademark) miRNA PCR Assays (manufactured by Qiagen, catalog No. 339306), SYBR (registered trademark) Green qPCR microRNA detection system (manufactured by Origin Technologies), and the like, and the kits can be used by designing a miRNA mutation-specific system.

The next generation sequencing (NGS) method can also be used as a method for more directly confirming and quantifying variants by comprehensively acquiring sequence information. The NGS method is a base sequence analysis method capable of analyzing base sequences in a test body in which extracts from a plurality of subjects are mixed in a massively parallel manner, and further capable of specifying which test body the base sequence is derived from by one analysis. Therefore, since one or more miRNA mutation-specific concentrations in a plurality of subjects can be quantified and determined, analysis can be performed more simply and more quickly. In the case of using the NGS method, MiSeq, NextSeq550, NovaSeq6000, or the like manufactured by Illumina, or a single-molecule sequencer manufactured by Pacific Biosciences, or the like can be used, but the NGS method is not limited thereto. The read information obtained by the next generation sequencer is aligned with, for example, a human genome full-length sequence, a miRNA sequence group, or a sequence of a target miRNA variant, and the number of reads is calculated, whereby the miRNA amount can be quantified and compared.

For the alignment, BWA, bowtie, bowtie2, or the like can be used, but the alignment is not limited thereto. When alignment is performed on the sequence of the miRNA variant of interest in a perfect match, reads having the same sequence as the miRNA variant can be quantified, and the number of reads can be used as a mutation-specific concentration. On the other hand, the mutation-specific concentration can also be calculated by aligning the wild type sequence group while allowing mismatches and extracting mutations. In that case, as a method for extracting the presence or absence of sequence variation from the reference sequence from the alignment information, the output of a mutation information storage file using bcftools or a mutation output program such as LoFreq or REDITools can be used, but the method is not limited thereto.

At the time of comparison between test bodies, the total number of aligned reads can be normalized by a TMM method, an RPKM normalization method, or the like and compared. For the analysis, a method of simply comparing the number of reads or the normalized value and performing a significant difference test, a platform for transcriptome analysis such as DESeq, EdgeR and the like, medical statistical software such as EZR (Easy R), JMP (registered trademark) and the like, or methods of creating a discriminant using Fisher's discriminant analysis, nonlinear discriminant analysis based on a Mahalanobis distance, logistic regression analysis, machine learning such as neural network, random forest and the like, can be used, but the method is not limited to the above methods.

In identifying between cancer and non-cancer, identification criteria such as concentration thresholds and the like may be determined and used.
For example, the threshold may be selected and changed according to the purpose of the inspection. For example, it is assumed that the setting will change in a case where it is desired to reduce the false negative rate as much as possible in order to prevent cancer subjects from overlooking, in a case where it is desired to reduce the false positive rate in order to make a definite diagnosis, or in other cases. Alternatively, it can be determined from an ROC (receiver operating characteristic) curve. The ROC curve plots (1-specificity) on the X-axis and sensitivity on the Y-axis, and it is located in the upper left corner in an ideal test (Sensitivity 100%, specificity 100%). The area under the ROC curve (area under the curve, AUC) can evaluate the usefulness of a quantitative test of a test, and is generally judged to have some performance of AUC0.7 or more. As the optimal threshold determined from the ROC curve, there are a case of using the Youden Index that selects the threshold that maximizes (sensitivity + specificity), and a minimum distance method that selects the threshold that minimizes the distance (= {(1-sensitivity)2 + (1-specificity)2}) from the upper left corner of the ROC curve, but are not limited thereto.

According to the fourth arrangement, it is possible to improve versatility by comparing mutation-specific concentrations. By using such a marker, it is less likely to be affected by a difference in miRNA extraction efficiency, a difference in tendency of increase/decrease in concentration, and variations among test bodies, which can occur among test bodies and data sets, and thus stable performance can be obtained.

### [EXAMPLES]

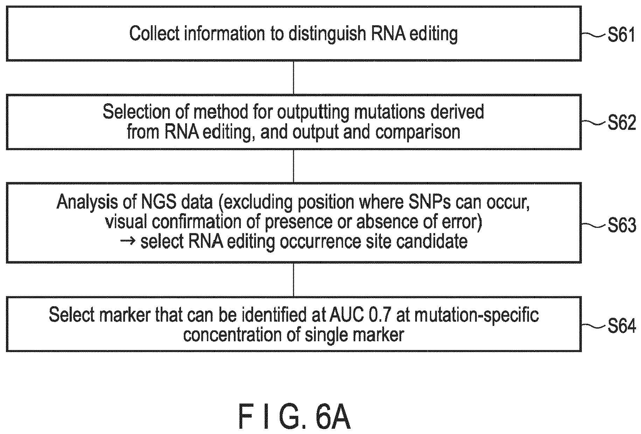

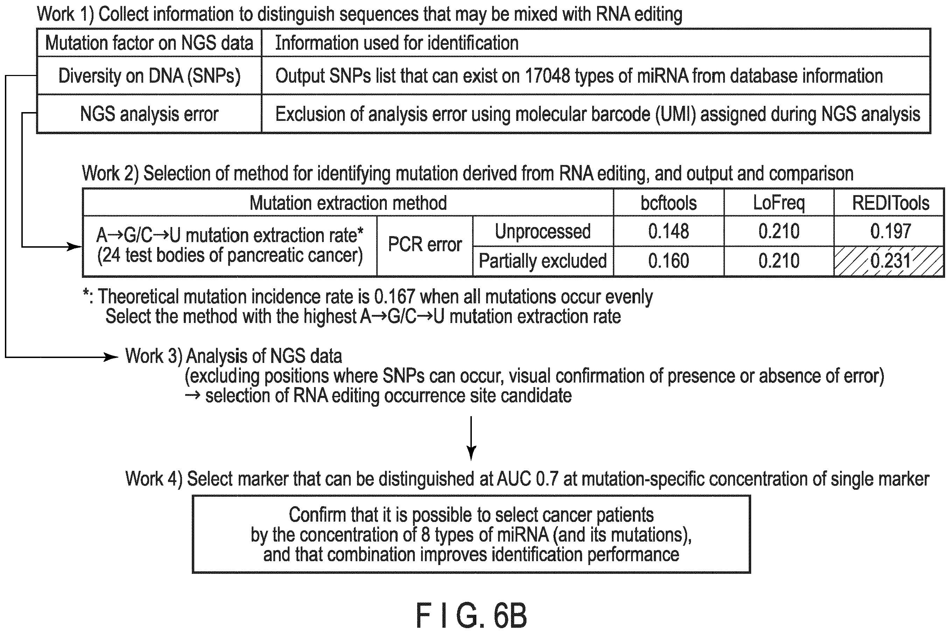

Hereinafter, experiments performed and data obtained thereby are shown. The rough progress of the experiment was made according to the working procedures shown in FIG. 6A and FIG. 6B. That is, as shown in FIG. 6A, first, information for distinguishing between diversity (SNP) on DNA and NGS analysis error and RNA editing was collected (S61), a method for outputting a mutation derived from RNA editing was selected, output and compared (S62), NGS data was analyzed (Visual check of presence or absence of error, excluding position where SNPs can occur), RNA editing occurrence point candidates were then selected (S63), and markers that can be identified with AUC0.7 at mutation-specific concentrations of single markers were selected (S64). Details of each operation are as described in FIG. 6B.

These specific steps will be described with reference to FIG. 6B. First, as work 1, in order to distinguish a sequence that can be mixed with RNA editing, first, mutation factors on NGS data, that is, diversity on DNA and an NGS analysis error were confirmed. With respect to diversity on DNA, existence of SNPs was identified by outputting an SNPs list that can exist on 17,048 types of miRNAs from database information. Regarding the NGS analysis error, an analysis error using a molecular barcode (UMI) assigned at the time of NGS analysis was identified, and those pieces of information were excluded (S61). Next, as work 2, selection and output of a method for identifying mutations derived from RNA editing were performed, and comparison was made. As a mutation extraction method, for 24 test bodies derived from pancreatic cancer patients, a method was selected in which the A → G/C → U mutation extraction rate was the highest, in which A was mutated to G and C was mutated to U. At this time, the theoretical mutation occurrence rate when all mutations are uniformly generated is 0.167. The PCR error was output for bcftools, LoFreq, and REDITools in an unprocessed case and a case in which a part was excluded. The respective results were 0.148 and 0.160, 0.210 and 0.210, 0.197 and 0.231 for the untreated and partially excluded bcftools, LoFreq and REDITool. From this result, it was found that the extraction rate of A → G/C → U mutation was the highest when REDITools was used and a part thereof was excluded, and data under this condition was used for the following analysis (S62). In the following work 3, NGS data was analyzed. Specifically, the presence or absence of an error excluding a portion (position) where SNPs can occur was visually confirmed. From the confirmed result, RNA editing occurrence part candidates were selected (S63). Next, as a work 4, a miRNA having a good AUC value at a mutation-specific concentration of a single marker was selected. For example, a marker that can be identified based on the vicinity of the AUC0.7 or the AUC0.7 or more was selected. The miRNAs thus selected were used in the following experiments (S64). Similarly, 24 test bodies derived from breast cancer patients were also analyzed.

### Example 1

A process of selecting an effective marker, in order to construct an identification system using the miRNA mutation-specific concentration in the serum of a non-cancer test body and a breast cancer/pancreatic cancer subject test body as an index, is described below. 2 times of NGS analysis were independently performed, and the number of test bodies was 24 test bodies of non-cancer test body serum, 24 test bodies of breast cancer, and 24 test bodies of pancreatic cancer (Table 3 NGS_DATA1, NGS_DATA2).

**Table 3: Number of test bodies used**

| Data set | NGS_DATA1 | NGS_DATA2 |
|---|---|---|
| Non-cancer | 24 | 24 |
| Breast cancer | 24 | 24 |
| Pancreatic cancer | 24 | 24 |
| Total | 72 | 72 |

The nucleic acid sequence in the serum was determined by next generation sequencer analysis. From 300 µL of all sera, miRNAs were extracted using miRNeasy Serum/Plasma Kit (Qiagen). Extraction of miRNAs was performed according to the protocol using QIAseq miRNA Library Kit (Qiagen) and QIAseq miRNA NGS 96 Index IL (Qiagen). A molecular barcode technology called UMI is used for the used index, and the influence of PCR duplicate and amplification bias due to gene amplification associated with library adjustment can be eliminated, and more accurate sequencing can be performed.

NGS analysis was performed using NovaSeq6000 (Single-ended, 75 bp) and data of 10 million or more reads was obtained for all test bodies. Using an extract command of UMI-tools (Genome Res. (2017) 27 (3): 491-499. PMID: 28100584), a FASTQ file from which the UMI has been removed was obtained. Further, QC based on read quality was performed. As the classification of sequences according to the type of miRNA, annotation for miRBase Release 22 was performed as Mismatch Allowable Parameter 2.

In addition, the sequence of miRBase Release 22 was defined as a wild type sequence. Alignment information (sam file) obtained by excluding the PCR duplicate from the obtained alignment information was output using the dedup command of the UMI-tools. The read number information aligned with each miRNA stored in the sam file was converted into a TMM value so that test bodies can be compared, and the TMM value was output as the concentration of each miRNA of interest including variants.

Mutation candidates were extracted from the sam file using REDITools. Only mutation candidates from A to G and from C to T were selected from the obtained mutation candidates, and mutation frequency information was obtained together with mutation sites. The TMM value of each miRNA of interest including the variant was multiplied by the obtained frequency information to obtain a mutation-specific concentration. In order to select a marker capable of identifying between a non-cancer test body and a breast cancer/pancreatic cancer test body, an ROC curve was created using the type of each test body (cancer/healthy subject) and the mutation-specific concentration of each obtained mutation candidate, and the AUC value of each was calculated and described in Table 4. Among the 20 candidate markers shown in Table 4, 8 markers shown in Table 2, that is, SEQ ID NO: 1 to NO: 8, showed an AUC value of 0.7 or more, indicating that the single marker can be used for identification of breast cancer/pancreatic cancer. In addition, SEQ ID NO: 9 to 13 has an AUC value of 0.6 or more, suggesting its availability.

**Table 4: Screening results of cancer marker candidates**

| No. | miRNA_ID_mu | Sequence_mu | Length | AUC values for single markers |
|---|---|---|---|---|
| 1 | 17_hsa-miR-199a-5p_C-T | CCCAGTGTTCAGACTATCTGTTC | 23 | 0.777 |
| 2 | 9_hsa-miR-1260b_A-G | ATCCCACCGCTGCCACCAT | 19 | 0.773 |
| 3 | 18_hsa-miR-146b-5p_A-G | TGAGAACTGAATTCCATGGGCTG | 23 | 0.766 |
| 4 | 9_hsa-miR-33b-5p_C-T | GTGCATTGTTGTTGCATTGC | 20 | 0.764 |
| 5 | 20_hsa-miR-15b-5p_A-G | TAGCAGCACATCATGGTTTGCA | 22 | 0.760 |
| 6 | 10_hsa-miR-92b-3p_C-T | TATTGCACTTGTCCCGGCCTCC | 22 | 0.746 |
| 7 | 12_hsa-miR-106a-5p_C-T | AAAAGTGCTTATAGTGCAGGTAG | 23 | 0.712 |
| 8 | 11_hsa-miR-98-5p_A-G | TGAGGTAGTAGGTTGTATTGTT | 22 | 0.705 |
| 9 | 13_hsa-miR-130a-3p_A-G | CAGTGCAATGTTGAAAGGGCAT | 22 | 0.689 |
| 10 | 11_hsa-miR-26a-5p_C-T | TTCAAGTAATTCAGGATAGGCT | 22 | 0.687 |
| 11 | 16_hsa-let-7d-5p_C-T | AGAGGTAGTAGGTTGTATAGTT | 22 | 0.644 |
| 12 | 17_hsa-let-7g-5p_A-G | TGAGGTAGTAGTTTGTGCAGTT | 22 | 0.614 |
| 13 | 9_hsa-miR-501-3p_C-T | AATGCACCTGGGCAAGGATTCT | 22 | 0.601 |
| 14 | 7_hsa-miR-335-3p_A-G | TTTTTCGTTATTGCTCCTGACC | 22 | 0.590 |
| 15 | 16_hsa-miR-320e-A-G | AAAGCTGGGTTGAGAGGG | 18 | 0.553 |
| 16 | 6_hsa-miR-1827_C-T | TGAGGTAGTAGATTGAAT | 18 | 0.544 |
| 17 | 10_hsa-miR-1273h-3p_C-T | CTGCAGACTTGACCTCCCAGGC | 22 | 0.542 |
| 18 | 10_hsa-miR-200a-3p_C-T | TAACACTGTTTGGTAACGATGT | 22 | 0.521 |
| 19 | 1_hsa-miR-99a-5p_A-G | GACCCGTAGATCCGATCTTGTG | 22 | 0.510 |
| 20 | 17_hsa-miR-4510_A-G | TGAGGGAGTAGGATGTGTGGTT | 22 | 0.504 |

### Examples 2 to 9

In order to examine whether breast cancer/pancreatic cancer can be more efficiently identified by combining the markers shown in Table 4, a discriminant using logistic regression analysis was created and the performance was evaluated. The test body of NGS_DATA1 shown in Table 3 was divided into two to obtain a test body for learning or test (Table 5).

**Table 5: Number of test bodies used in Examples 5 to 12**

| Data set | NGS_DATA1 | |
|---|---|---|
| Test body | Training | Test |
| Non-cancer | 12 | 12 |
| Breast cancer | 12 | 12 |
| Pancreatic cancer | 12 | 12 |
| Total | 36 | 36 |

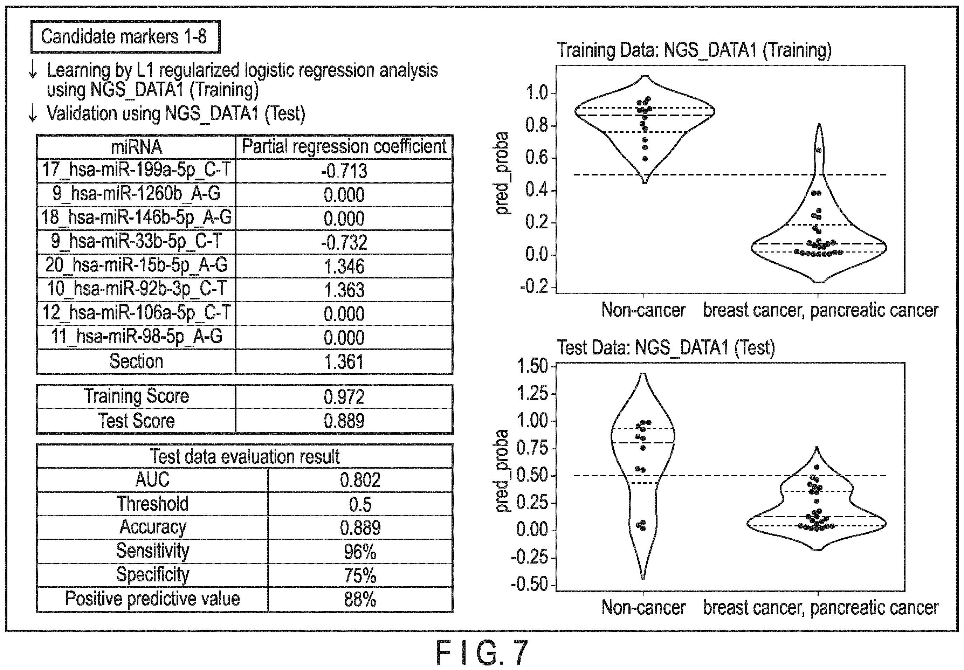

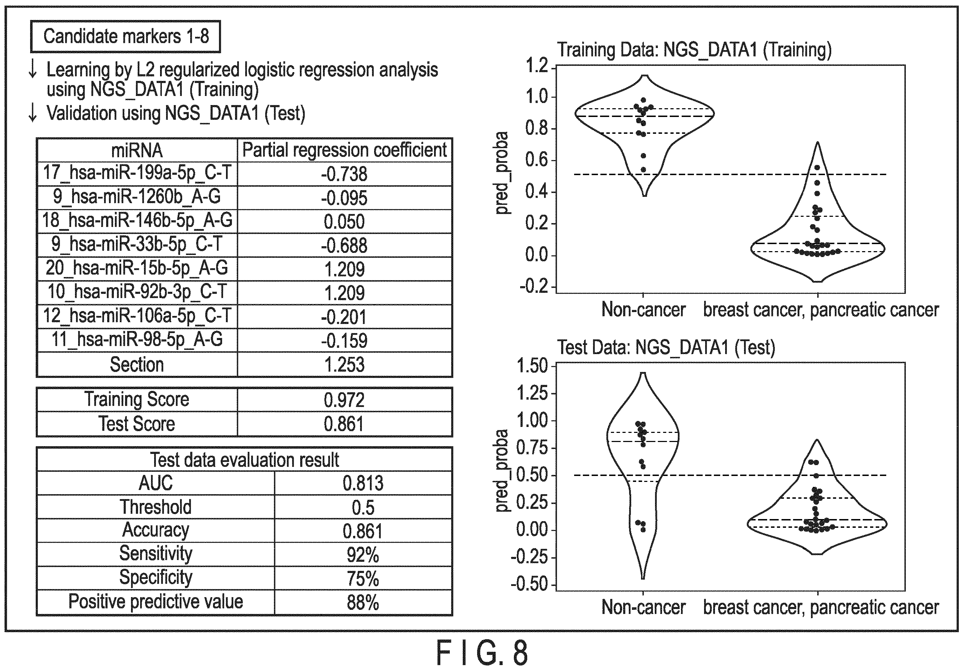

As a result of logistic regression analysis using L1 regularization, the identification performance of the test body for test was AUC0.802 by using 4 kinds of markers except for the marker having the coefficient of 0 among the 8 markers, and the identification performance was higher than that when the markers were used alone (Example 2, FIG. 7). When the threshold was set to 0.5, the sensitivity was 96%, the specificity was 75%, and the positive predictive value was 88%, and it was shown that high performance that can be used for a cancer identification test was achieved. When all 8 markers were used in the logistic regression analysis using L2 regularization, the identification performance of the test body for test was AUC0.813, which was higher than the identification performance when used alone (Example 3, FIG. 8). When the threshold was set to 0.5, the sensitivity was 92%, the specificity was 75%, and the positive predictive value was 88%, and similar excellent results were obtained.

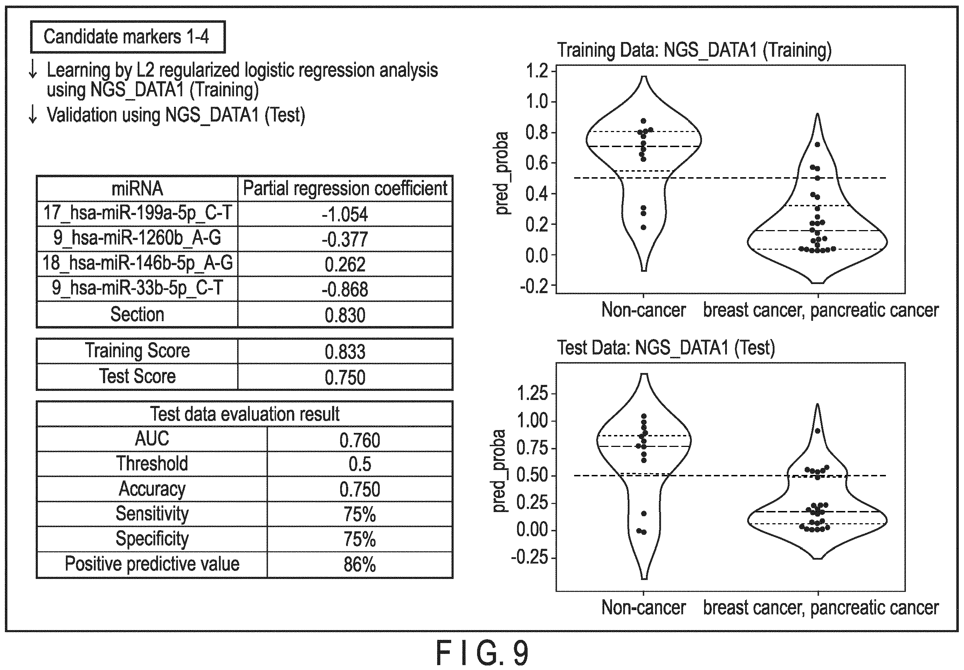

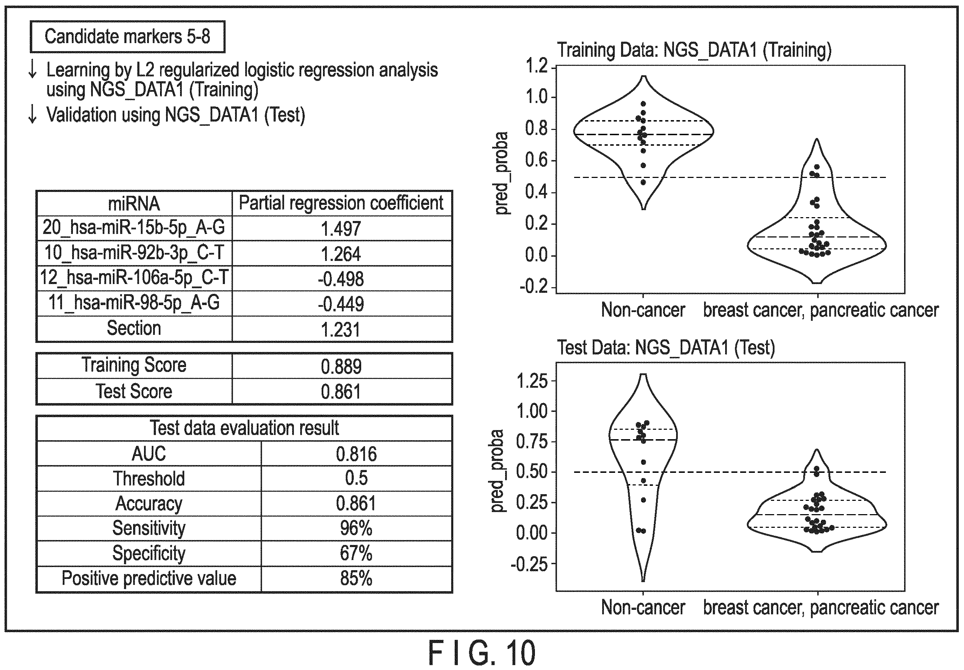

In the arrangement, 0.5 is used as a threshold for indicating the reference performance. In logistic regression analysis using L2 regularization, when only markers 1 to 4 in Table 4 were used (Example 4, FIG. 9), and when only markers 5 to 8 were used (Example 5, FIG. 10), similarly high identification performance was exhibited, and AUC0.760 and AUC0.816, respectively, showed higher identification performance than when used alone (FIGS. 9 and 10). In addition, when the threshold was set to 0.5, the sensitivity was 75% and 96%, the specificity was 75% and 67%, and the positive predictive value was 86% and 85%, indicating that these markers have higher identification performance by being combined.

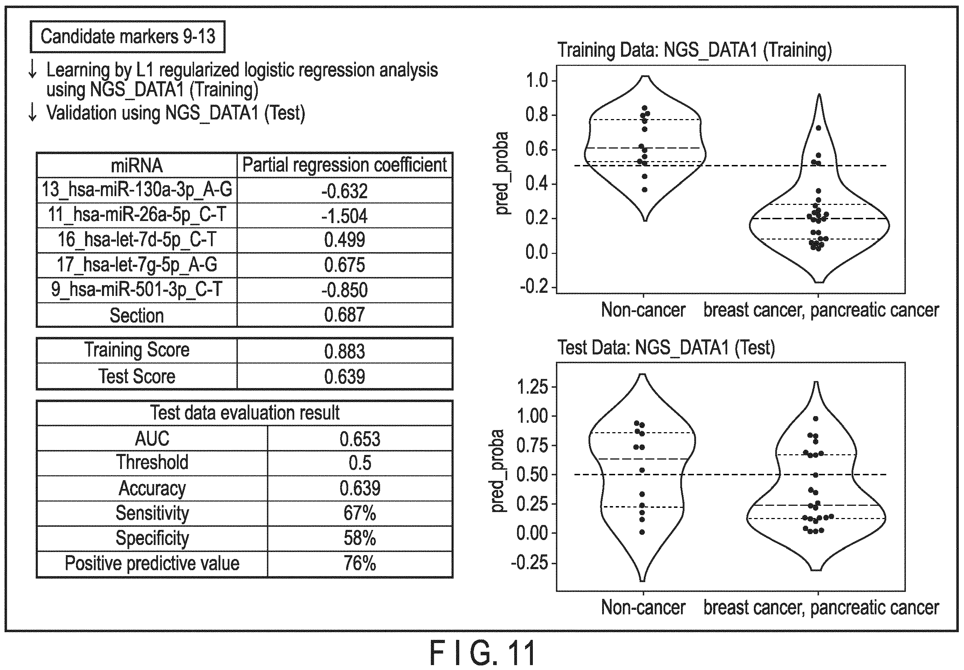

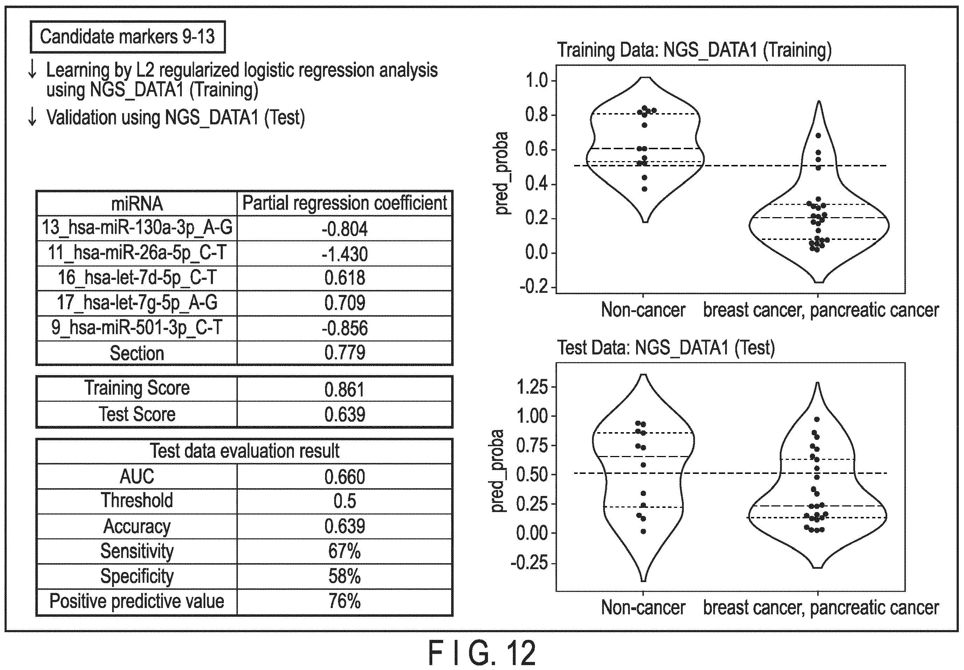

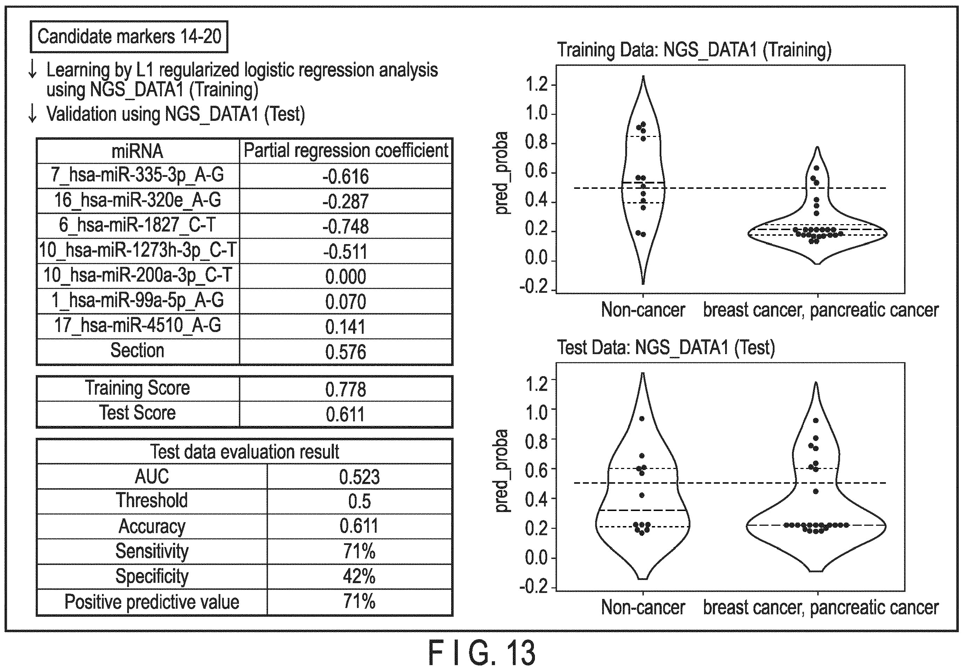

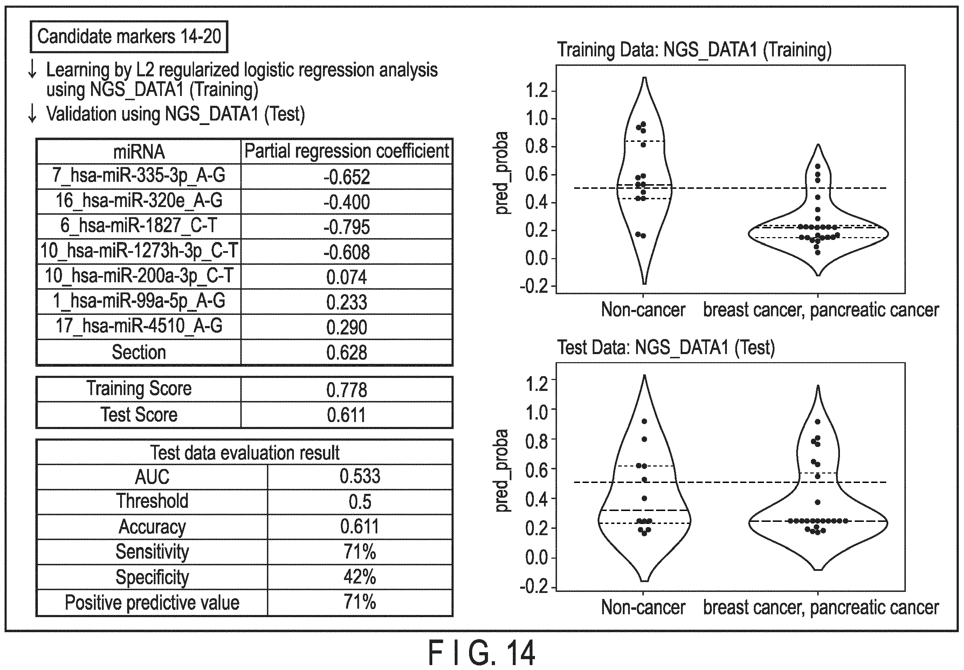

On the other hand, in the case of No. 9 to No. 13 (Examples 6 and 7, FIGS. 11 and 12) in Table 4 in which the identification performance was not shown alone, the AUC values were 0.653 and 0.660, respectively, which did not reach the above example. When No. 14 to No. 20 (Examples 8 and 9 and FIGS. 13 and 14) were used, the AUC values were as low as 0.523 and 0.533, respectively, and hardly showed identification performance.

From the above results, it was shown that the markers shown in Table 2 are more useful markers.

### Examples 10 to 17

A general cancer subject identification system using miRNA concentrations does not focus on mutation-specific concentrations, and uses miRNA concentrations including mutations as the concentration of all miRNAs having diversity. To confirm the advantages of using the mutation-specific concentrations shown in the present system, a reproducibility evaluation between the data sets was performed. In order to verify whether the discriminant can be applied to the data set (Table 3 NGS_DATA2) acquired using different devices (NextSeq500 instead of NovaSeq6000, other approaches are identical) at different times, similar verification was performed with the learning data as NGS_DATA1 and the test data as NGS_DATA2 (Table 6). Note that, in order to evaluate the effectiveness of using a mutation-specific concentration, the same learning and test were performed using the total concentration (TMM value) including variants of the same miRNA.

**Table 6: Number of test bodies used in Examples 13 to 20**

| Data set | NGS_DATA1 | NGS_DATA2 |
|---|---|---|
| Test body | Training | Test |
| Non-cancer | 24 | 24 |
| Breast cancer | 24 | 24 |
| Pancreatic cancer | 24 | 24 |
| Total | 72 | 72 |

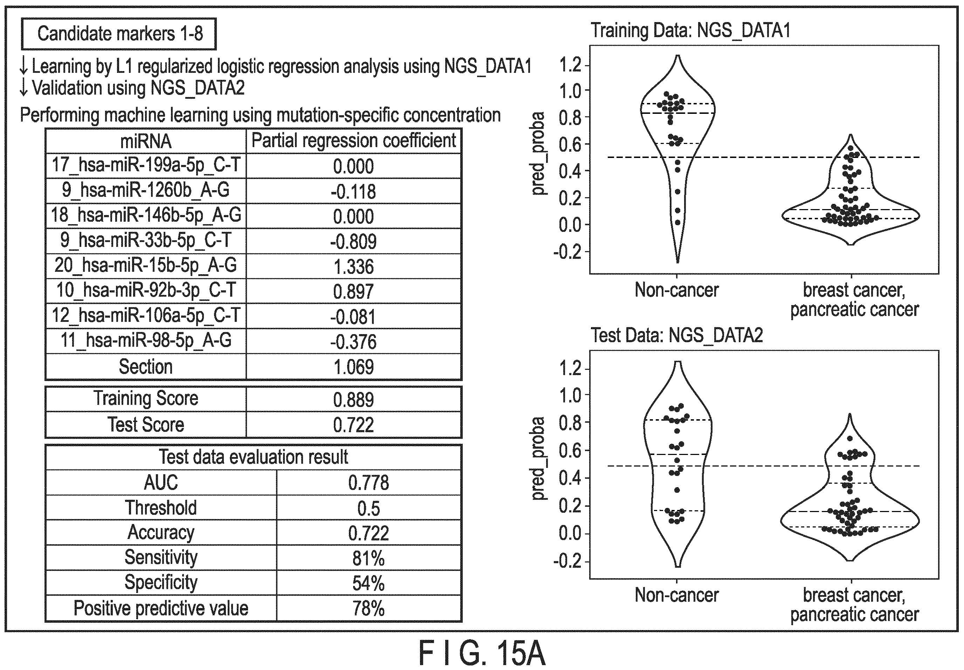

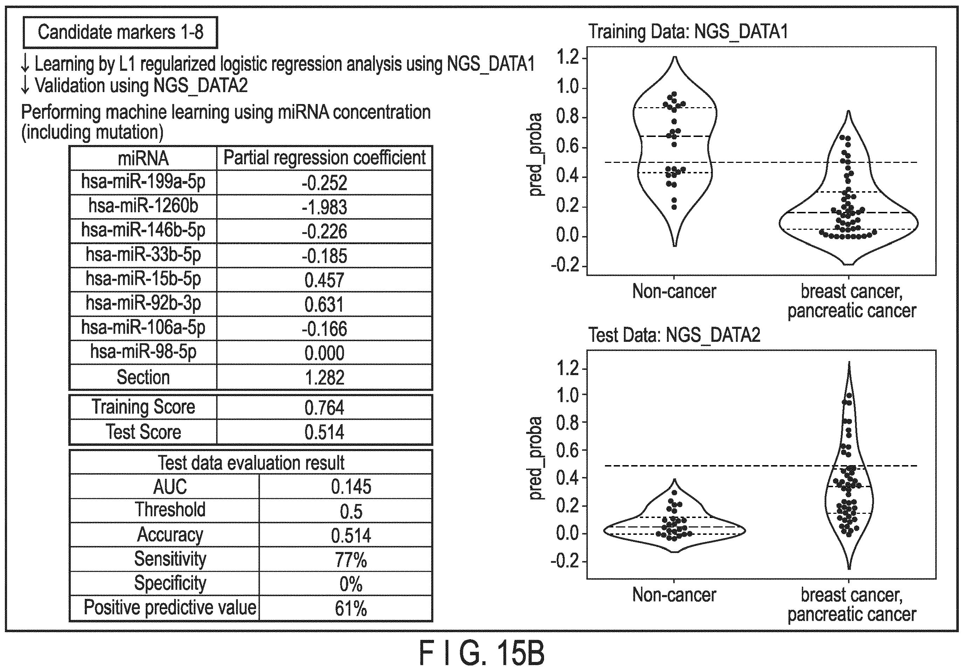

As a result of logistic regression analysis using L1 regularization using the marker 1 to 8 in Table 4, the AUC value was as high as 0.778 when a mutation-specific concentration was used (Example 10, FIG. 15A). On the other hand, in a case where all concentrations including variants were used, the magnitude of the output value from the discriminant was reversed between the learning data and the test data, and it was suggested that the AUC value was 0.145 and identification performance was not exhibited (Example 11, FIG. 15B).

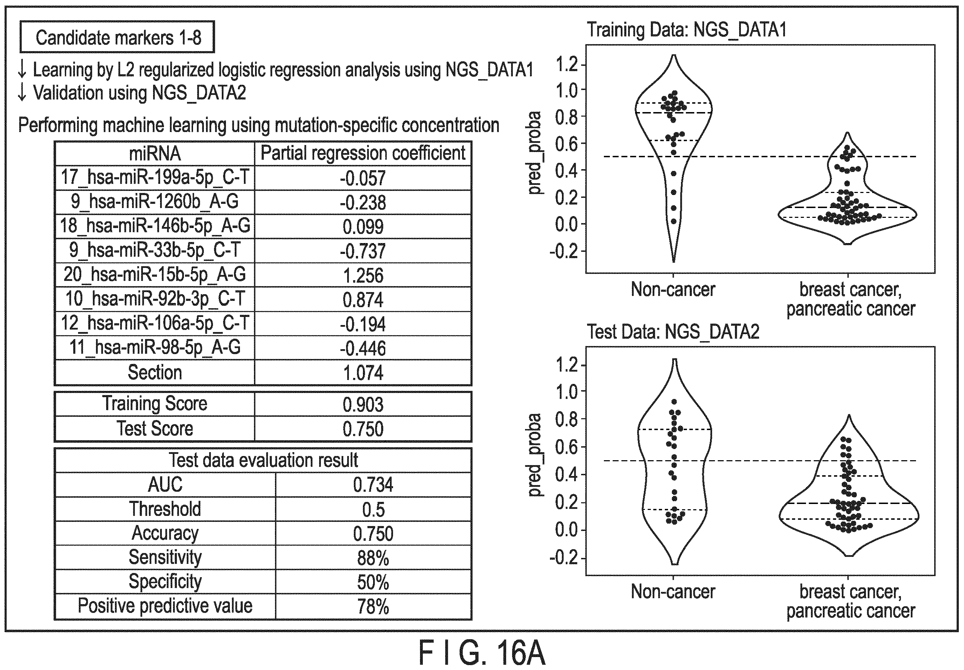

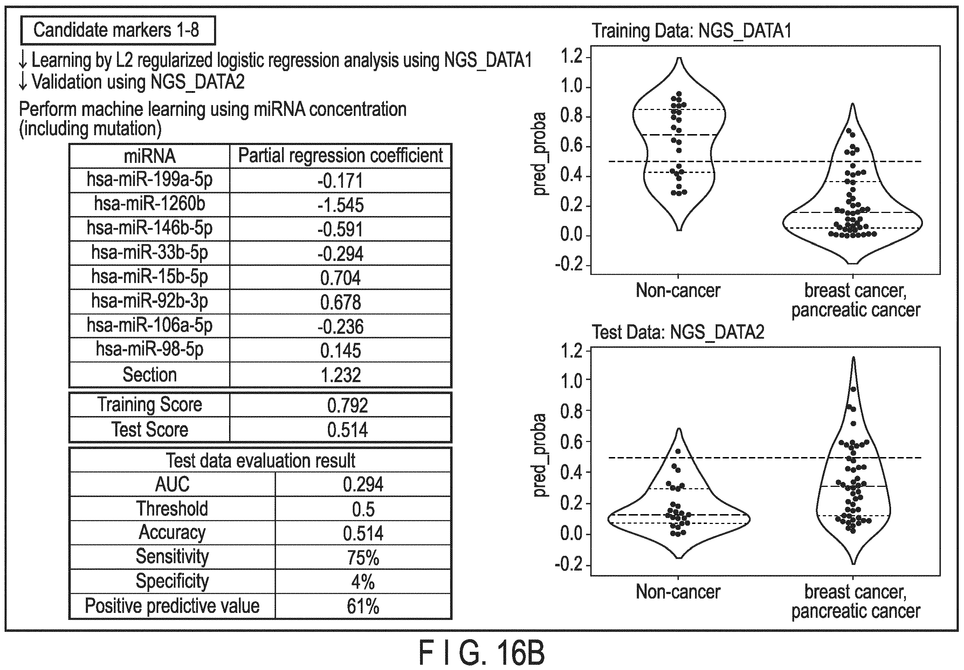

A similar tendency was also confirmed by logistic regression analysis using L2 regularization. When a mutation-specific concentration was used, the AUC value was as high as 0.734 (Example 12, FIG. 16A). On the other hand, in a case where all concentrations including variants were used, the magnitude of the output value from the discriminant was reversed between the learning data and the test data, and the AUC value was as low as 0.294, suggesting that the identification performance was not exhibited (Example 13, FIG. 16B).

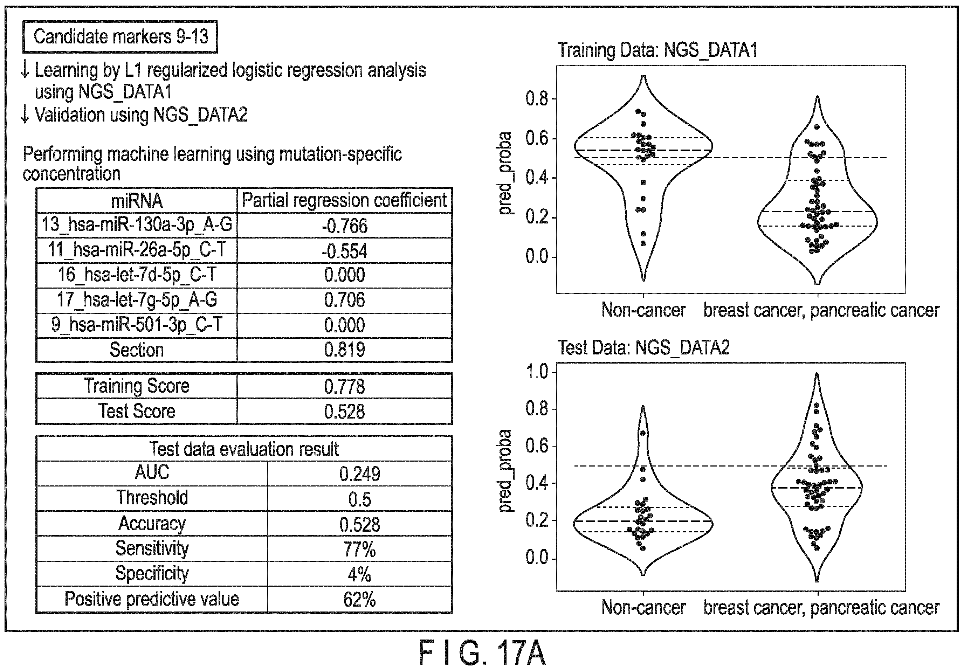

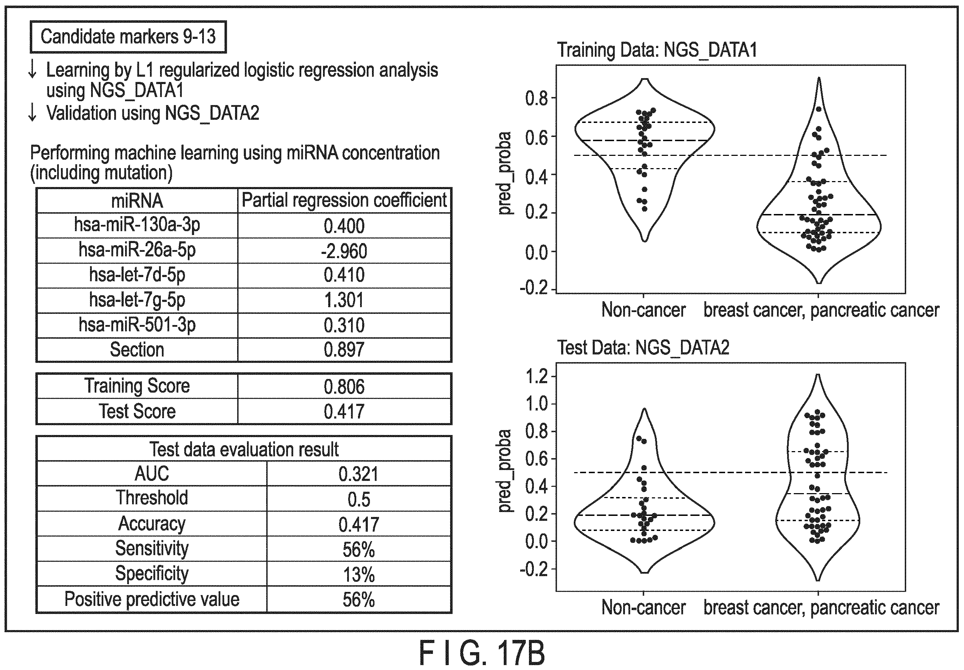

As a result of performing logistic regression analysis using L1 regularization using the marker No. 9 to 13 in Table 4, the AUC value was 0.249 (Example 14, FIG. 17A) when the mutation-specific concentration was used, while the identification performance was low together with the AUC value of 0.321 (Example 15, FIG. 17B) even when all the concentrations including the variants were used.

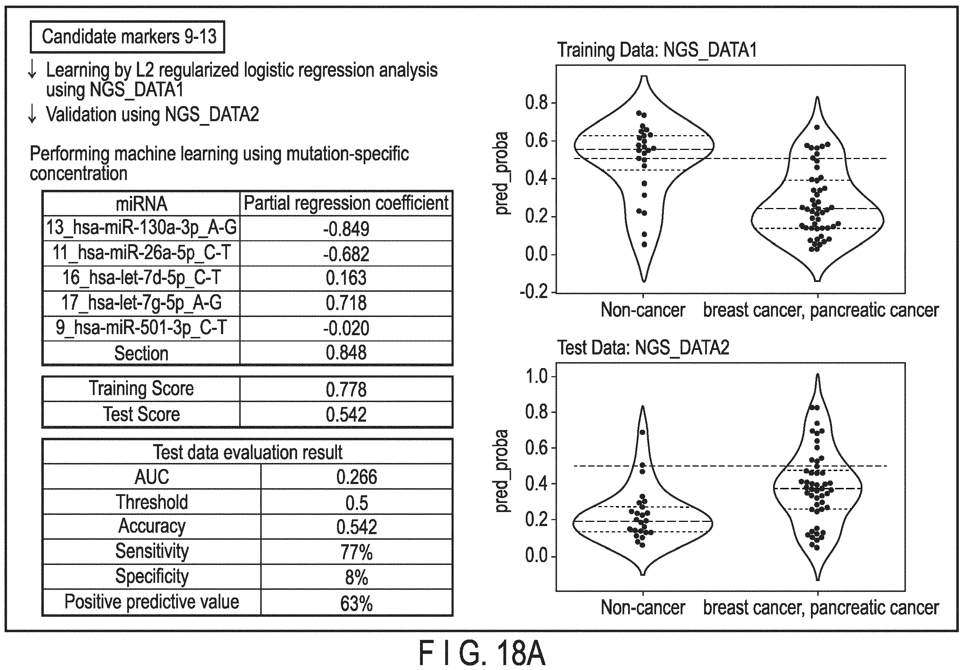

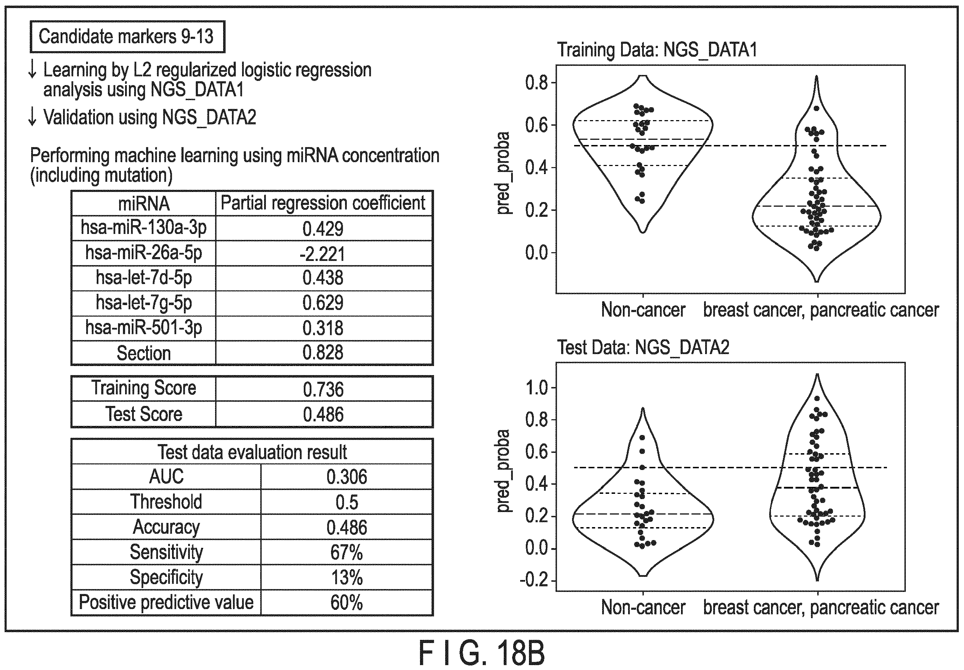

Similar results were confirmed in logistic regression analysis using L2 regularization for the same marker No. 9 to 13. When the mutation-specific concentration was used, the AUC value was 0.266 (Example 16, FIG. 18A), and when the total concentration was used, the AUC value was 0.306 (Example 17, FIG. 18B), and the identification performance was low.

According to the above arrangement, a method for identifying the feature of a test body and a miRNA cancer marker set having stable detection performance are provided.

As a further arrangement, the following method may also be provided.
- A method for identifying between a cancer subject and a healthy subject, the method comprising measuring a mutation-specific concentration of at least one type of miRNA contained in a test body;
   correcting the mutation-specific concentration to obtain a corrected mutation-specific concentration;
   and determining whether the test body is a cancer test body or a non-cancer test body, using an increase or decrease in the corrected mutation-specific concentration obtained as an index.
- A method for identifying between a cancer subject and a healthy subject, the method comprising measuring a mutation-specific concentration of at least one type of miRNA contained in a test body derived from a subject;
   correcting the mutation-specific concentration to obtain a corrected mutation-specific concentration;
   and determining a possibility that a subject has cancer or a risk of developing cancer using an increase or decrease in the obtained corrected mutation-specific concentration as an index.

Here, all are described as DNA sequences for nucleic acids. As described above, in the sequence set forth in SEQ ID NO: 1 to 20, "T" can be replaced with "U", and any nucleic acid indicated thereby is included in the range according to any of the present arrangements. However, this paragraph describes, for reference, sequence groups that represent "T" as "U";
17_hsa-miR-199a-5p_C-U SEQ ID NO: 21 (corresponding to SEQ ID NO: 1)CCCAGUGUUC AGACUAUCUG UUC 23;
9_hsa-miR-1260b_A-G SEQ ID NO: 22 (corresponding to SEQ ID NO: 2)AUCCCACCGC UGCCACCAU 19;
18_hsa-miR-146b-5p_A-G SEQ ID NO: 23 (corresponding to SEQ ID NO: 3)UGAGAACUGA AUUCCAUGGG CUG 23;
9_hsa-miR-33b-5p_C-U SEQ ID NO: 24 (corresponding to SEQ ID NO: 4)GUGCAUUGUU GUUGCAUUGC 20;
20_hsa-miR-15b-5p_A-G SEQ ID NO: 25 (corresponding to SEQ ID NO: 5)UAGCAGCACA UCAUGGUUUG CA 22;
10_hsa-miR-92b-3p_C-U SEQ ID NO: 26 (corresponding to SEQ ID NO: 6)UAUUGCACUU GUCCCGGCCU CC 22;
12_hsa-miR-106a-5p_C-U SEQ ID NO: 27 (corresponding to SEQ ID NO: 7)AAAAGUGCUU AUAGUGCAGG UAG 23;
11_hsa-miR-98-5p_A-G SEQ ID NO: 28 (corresponding to SEQ ID NO: 8)UGAGGUAGUA GGUUGUAUUG UU 22;
13_hsa-miR-130a-3 p_A-G SEQ ID NO: 29 (corresponding to SEQ ID NO: 9)CAGUGCAAUG UUGAAAGGGC AU 22;
11_hsa-miR-26a-5p_C-U SEQ ID NO: 30 (corresponding to SEQ ID NO: 10)UUCAAGUAAU UCAGGAUAGG CU 22;
16_hsa-let-7d-5p_C-U SEQ ID NO: 31 (corresponding to SEQ ID NO: 11)AGAGGUAGUA GGUUGUAUAG UU 22;
17_hsa-let-7g-5p_A-G SEQ ID NO: 32 (corresponding to SEQ ID NO: 12)UGAGGUAGUA GUUUGUGCAG UU 22;
9_hsa-miR-501-3p_C-U SEQ ID NO: 33 (corresponding to SEQ ID NO: 13)AAUGCACCUG GGCAAGGAUU CU 22;
7_hsa-miR-335-3p_A-G SEQ ID NO: 34 (corresponding to SEQ ID NO: 14)UUUUUCGUUA UUGCUCCUGA CC 22;
16_hsa-miR-320e_A-G SEQ ID NO: 35 (corresponding to SEQ ID NO: 15)AAAGCUGGGU UGAGAGGG 18;
6_hsa-miR-1827_C-U SEQ ID NO: 36 (corresponding to SEQ ID NO: 16)UGAGGUAGUA GAUUGAAU 18;
10_hsa-miR-1273h-3p_C-U SEQ ID NO: 37 (corresponding to SEQ ID NO: 17)CUGCAGACUU GACCUCCCAG GC 22;
10_hsa-miR-200a-3p_C-U SEQ ID NO: 38 (corresponding to SEQ ID NO: 18)UAACACUGUU UGGUAACGAU GU 22;
1_hsa-miR-99a-5p_A-G SEQ ID NO: 39 (corresponding to SEQ ID NO: 19)GACCCGUAGA UCCGAUCUUG UG 22;
17_hsa-miR-4510_A-G SEQ ID NO: 40 (corresponding to SEQ ID NO: 20)UGAGGGAGUA GGAUGUGUGG UU 22.

While certain arrangements have been described, these arrangements have been presented by way of example only, and are not intended to limit the scope of the claims. Indeed, the methods described herein may be embodied in a variety of other forms; furthermore, various omissions, substitutions and changes in the form of the methods described herein may be made.

The arrangements as described above include clauses below.

### Clause 1

A method of identifying a feature of a test body, characterized by comprising:
measuring a mutation-specific concentration of at least one type of miRNA contained in the test body;
correcting a value of the mutation-specific concentration in order to standardize data and acquiring the corrected mutation-specific concentration; and
determining whether the test body is a cancer test body or a non-cancer test body using an increase or decrease in the obtained corrected mutation-specific concentration as an index.

### Clause 2

The method according to Clause 1, characterized in that the miRNA is at least one type of miRNA represented by any one of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 and SEQ ID NO: 13, or a sequence including one or several substitutions or deletions, or one or several additions at a site, or a corresponding site to the site, other than a characterizing site that characterizes a wild type and mutant type of interest of each of these sequences, and a complementary sequence thereof.

### Clause 3

The method according to Clause 1, characterized in that the miRNA is at least one type of miRNA represented by any one of SEQ ID NO: 1, SEQ ID NO: 2, SEQ ID NO: 3, SEQ ID NO: 4, SEQ ID NO: 5, SEQ ID NO: 6, SEQ ID NO: 7 and SEQ ID NO: 8, or a sequence including one or several substitutions, deletions, or one or several additions at a site, or a corresponding site to the site, other than a characterizing site that characterizes a wild type or mutant type of interest of each sequence, and a complementary sequence thereof.

### Clause 4

The method according to one of Clause 1 to Clause 3, characterized in that the correction to the mutation-specific concentration is performed by multiplying a normalized value of a total number of aligned reads by frequency information or dividing by an internal standard, depending on the method of measuring miRNA.

### Clause 5

The method according to Clause 4, characterized in that the increase or decrease in the resulting corrected mutation-specific concentration is determined by a predetermined threshold.

### Clause 6

The method according to one of Clause 1 to Clause 3, characterized in that the mutation is a 1 base substitution.

### Clause 7

The method according to one of Clause 1 to Clause 3, characterized in that the test body is serum or plasma.

### Clause 8

The method according to one of Clause 1 to Clause 3, characterized in that the cancer is at least one cancer selected from the group consisting of breast cancer and pancreatic cancer.

### Clause 9

he method according to one of Clause 1 to Clause 3, characterized in that the measuring is performed by an NGS method, a PCR method, or a microarray method.

### Clause 10

A cancer marker set, characterized by comprising a group of miRNAs each having SEQ ID NO: 1, SEQ ID NO: 2, SEQ ID NO: 3, SEQ ID NO: 4, SEQ ID NO: 5, SEQ ID NO: 6, SEQ ID NO: 7, and SEQ ID NO: 8, or a sequence including one or several substitutions, deletions, or one or several additions at a site, or a corresponding site to the site, other than a site that characterizes the wild type and mutant type of interest of each of these sequences, or a complementary sequence thereof.

### Clause 11

A miRNA cancer marker, characterized by comprising a sequence selected from a group consisting of SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8, and a complementary sequence thereof.

### Clause 12

A miRNA cancer marker, characterized by comprising a miRNA selected from a group consisting of 17_hsa-miR-199a-5p_C-T, 9_hsa-miR-1260b_A-G, 18_hsa-miR-146b-5p_A-G, 9_hsa-miR-33b-5p_C-T, 20_hsa-miR-15b-5p_A-G, 10_hsa-miR-92b-3p_C-T, 12_hsa-miR-106a-5p_C-T, 11_hsa-miR-98-5p_A-G, 13_hsa-miR-130 a-3p_A-G, 11_hsa-miR-26a-5p_C-T, 16_hsa-let-7d-5p_C-T, 17_hsa-let-7g-5p_A-G and 9_hsa-miR-501-3p_C-T.

### Clause 13

A miRNA cancer marker set, characterized by comprising a group of miRNAs each being17_hsa-miR-199a-5p_C-T, 9_hsa-miR-1260b_A-G, 18_hsa-miR-146b-5p_A-G, 9_hsa-miR-33b-5p_C-T, 20_hsa-miR-15b-5p_A-G, 10_hsa-miR-92b-3p_C-T, 12_hsa-miR-106a-5p_C-T, 11_hsa-miR-98-5p_A-G, 13_hsa-miR-130 a-3p_A-G, 11_hsa-miR-26a-5p_C-T, 16_hsa-let-7 d-5p_C-T, 17_hsa-let-7 g-5p_A-G and 9_hsa-miR-501-3p_C-T.

### Clause 14

A miRNA cancer marker, characterized by comprising a miRNA selected from a group consisting of 17_hsa-miR-199a-5p_C-T, 9_hsa-miR-1260b_A-G, 18_hsa-miR-146b-5p_A-G, 9_hsa-miR-33b-5p_C-T, 20_hsa-miR-15b-5p_A-G, 10_hsa-miR-92b-3p_C-T, 12_hsa-miR-106a-5p_C-T, and 11_hsa-miR-98-5p_A-G.

### Clause 15

A miRNA cancer marker set, characterized by comprising a group of miRNAs each being 17_hsa-miR-199a-5p_C-T, 9_hsa-miR-1260b_A-G, 18_hsa-miR-146b-5p_A-G, 9_hsa-miR-33b-5p_C-T, 20_hsa-miR-15b-5p_A-G, 10_hsa-miR-92b-3p_C-T, 12_hsa-miR-106a-5p_C-T, and 11_hsa-miR-98-5p_A-G.

## Claims

1. A method of identifying a feature of a test body, **characterized by** comprising:
measuring a mutation-specific concentration of at least one type of miRNA contained in the test body;
correcting a value of the mutation-specific concentration in order to standardize data and acquiring the corrected mutation-specific concentration; and
determining whether the test body is a cancer test body or a non-cancer test body using an increase or decrease in the obtained corrected mutation-specific concentration as an index.

2. The method according to claim 1, **characterized in that** the miRNA is at least one type of miRNA represented by any one of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 and SEQ ID NO: 13, or a sequence including one or several substitutions or deletions, or one or several additions at a site, or a corresponding site to the site, other than a characterizing site that characterizes a wild type and mutant type of interest of each of these sequences, and a complementary sequence thereof.

3. The method according to claim 1, **characterized in that** the miRNA is at least one type of miRNA represented by any one of SEQ ID NO: 1, SEQ ID NO: 2, SEQ ID NO: 3, SEQ ID NO: 4, SEQ ID NO: 5, SEQ ID NO: 6, SEQ ID NO: 7 and SEQ ID NO: 8, or a sequence including one or several substitutions, deletions, or one or several additions at a site, or a corresponding site to the site, other than a characterizing site that characterizes a wild type or mutant type of interest of each sequence, and a complementary sequence thereof.

4. The method according to one of claims 1 to 3, **characterized in that** the correction to the mutation-specific concentration is performed by multiplying a normalized value of a total number of aligned reads by frequency information or dividing by an internal standard, depending on the method of measuring miRNA.

5. The method according to claim 4, **characterized in that** the increase or decrease in the resulting corrected mutation-specific concentration is determined by a predetermined threshold.

6. The method according to one of claims 1 to 3, **characterized in that** the mutation is a 1 base substitution.

7. The method according to one of claims 1 to 3, **characterized in that** the test body is serum or plasma.

8. The method according to one of claims 1 to 3, **characterized in that** the cancer is at least one cancer selected from the group consisting of breast cancer and pancreatic cancer.

9. The method according to one of claims 1 to 3, **characterized in that** the measuring is performed by an NGS method, a PCR method, or a microarray method.

10. A cancer marker set, **characterized by** comprising a group of miRNAs each having SEQ ID NO: 1, SEQ ID NO: 2, SEQ ID NO: 3, SEQ ID NO: 4, SEQ ID NO: 5, SEQ ID NO: 6, SEQ ID NO: 7, and SEQ ID NO: 8, or a sequence including one or several substitutions, deletions, or one or several additions at a site, or a corresponding site to the site, other than a site that characterizes the wild type and mutant type of interest of each of these sequences, or a complementary sequence thereof.

11. A miRNA cancer marker according to claim 10, **characterized by** comprising a sequence selected from a group consisting of SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8, and a complementary sequence thereof.

12. A miRNA cancer marker according to claim 10, **characterized by** comprising a miRNA selected from a group consisting of 17_hsa-miR-199a-5p_C-T, 9_hsa-miR-1260b_A-G, 18_hsa-miR-146b-5p_A-G, 9_hsa-miR-33b-5p_C-T, 20_hsa-miR-15b-5p_A-G, 10_hsa-miR-92b-3p_C-T, 12_hsa-miR-106a-5p_C-T, 11_hsa-miR-98-5p_A-G, 13_hsa-miR-130 a-3p_A-G, 11_hsa-miR-26a-5p_C-T, 16_hsa-let-7d-5p_C-T, 17_hsa-let-7g-5p_A-G and 9_hsa-miR-501-3p_C-T.

13. A miRNA cancer marker set according to claim 10, **characterized by** comprising a group of miRNAs each being 17_hsa-miR-199a-5p_C-T, 9_hsa-miR-1260b_A-G, 18_hsa-miR-146b-5p_A-G, 9_hsa-miR-33b-5p_C-T, 20_hsa-miR-15b-5p_A-G, 10_hsa-miR-92b-3p_C-T, 12_hsa-miR-106a-5p_C-T, 11_hsa-miR-98-5p_A-G, 13_hsa-miR-130 a-3p_A-G, 11_hsa-miR-26a-5p_C-T, 16_hsa-let-7 d-5p_C-T, 17_hsa-let-7 g-5p_A-G and 9_hsa-miR-501-3p_C-T.

14. A miRNA cancer marker according to claim 10, **characterized by** comprising a miRNA selected from a group consisting of 17_hsa-miR-199a-5p_C-T, 9_hsa-miR-1260b_A-G, 18_hsa-miR-146b-5p_A-G, 9_hsa-miR-33b-5p_C-T, 20_hsa-miR-15b-5p_A-G, 10_hsa-miR-92b-3p_C-T, 12_hsa-miR-106a-5p_C-T, and 11_hsa-miR-98-5p_A-G.

15. A miRNA cancer marker set according to claim 10, **characterized by** comprising a group of miRNAs each being 17_hsa-miR-199a-5p_C-T, 9_hsa-miR-1260b_A-G, 18_hsa-miR-146b-5p_A-G, 9_hsa-miR-33b-5p_C-T, 20_hsa-miR-15b-5p_A-G, 10_hsa-miR-92b-3p_C-T, 12_hsa-miR-106a-5p_C-T, and 11_hsa-miR-98-5p_A-G.
